(19) Europäisches Patentamt
European Patent Office
Office européen des brevets

(11) EP 1 331 263 A1

(12) EUROPEAN PATENT APPLICATION
published in accordance with Art. 158(3) EPC

(43) Date of publication:
30.07.2003 Bulletin 2003/31

(21) Application number: 01972647.0

(22) Date of filing: 02.10.2001

(51) Int Cl.[7]: C12N 1/18, A21D 8/04
// C12N1:18, C12R1:865

(86) International application number:
PCT/JP01/08668

(87) International publication number:
WO 02/031118 (18.04.2002 Gazette 2002/16)

(84) Designated Contracting States:
AT BE CH CY DE DK ES FI FR GB GR IE IT LI LU MC NL PT SE TR

(30) Priority: 02.10.2000 JP 2000302166
02.10.2000 JP 2000302167
02.10.2000 JP 2000302168
02.10.2000 JP 2000302169
06.10.2000 JP 2000307267
06.10.2000 JP 2000307268
31.05.2001 JP 2001165097

(71) Applicant: KANEKA CORPORATION
Osaka-shi, Osaka 530-8288 (JP)

(72) Inventors:
- KATSUMI, Toshiaki
Himeji-shi, Hyogo 671-0254 (JP)
- OHTSUKI, Kinya
Nishinomiya-shi, Hyogo 662-0026 (JP)
- TASHITA, Yasuhiro
Kobe-shi, Hyogo 653-0854 (JP)

(74) Representative: VOSSIUS & PARTNER
Siebertstrasse 4
81675 München (DE)

# (54) DRYING-RESISTANT YEAST

(57) The present invention provides to an yeast having drying tolerance, which is suitable for producing bread, especially for producing bread with a frozen dough, a dry yeast produced by drying the yeast, a dough comprising the above-mentioned yeast or the above-mentioned dry yeast, and bread produced by using the dough.



Printed by Jouve, 75001 PARIS (FR)

## Description

### TECHNICAL FIELD

The present invention relates to an yeast having drying tolerance, which is suitable for producing bread, especially for producing bread from frozen dough, a dry yeast produced by drying the yeast, a dough comprising the above-mentioned yeast or the above-mentioned dry yeast, and bread produced from the dough.

### BACKGROUND ART

The yeasts are largely grouped into two kinds of forms of raw yeasts (which may be hereinafter referred to as "yeast before drying" in some cases) and dry yeasts (which may be hereinafter referred to as "yeast after drying" in some cases). As the raw yeast, there have been developed yeasts having various actions such as an yeast usable in a dough having a high sugar concentration, an yeast usable in a frozen dough process and an yeast having low-temperature sensitivity, and yeasts which are suitable for production of desired bread have been actually used, which can meet the needs for production of various breads. However, the raw yeast must be stored with refrigeration, and its storage period of time is short.

On the other hand, a dry yeast is one produced by drying a raw yeast for the purposes of improving storage stability and preservability, and has been actually used as active dry yeasts and instant dry yeasts. Furthermore, the flavor and taste peculiar to dry yeasts have been preferred in a bread having a low sugar concentration, and actually used. In the production of the dry yeast, it is necessary to use a strain having drying tolerance, or to give drying tolerance to an yeast by adjusting the cultivation method. Also, in drying, it is necessary, for instance, to come up with a means of a drying apparatus or to come up with a means of temperature or to add an emulsifier. As described above, in the production of the dry yeast manufactured article, it has been difficult to produce dry yeast product in which the performance of the yeast is prevented from lowering as much as possible during drying, with keeping the actions of the yeast such as leavening ability to the same extent as those of the raw yeast, so that the actions of the dry yeast cannot be said to be satisfactory as compared to those of the raw yeast. Therefore, there have been desired to realize a dry yeast having excellent preservability and storage stability, and having various actions to the same extent as those in the raw yeast.

At present, the dry yeast has been mainly used in the baking process under a limited sugar concentration in which the flavor and taste peculiar to the dry yeast are especially favored. Concretely, the dry yeast is mainly used in the production of French bread produced by a scratch process, and the production of bread having a low sugar concentration such as white bread. On the other hand, the dry yeast has been little used in a process for producing bread using a dough having a high sugar concentration, or a frozen dough process using a frozen dough or a dough stored by refrigeration. This is due to the fact that an yeast having a satisfactory property for producing bread has not yet been found in these doughs. As an yeast which can be used as a dry yeast in a process of producing a frozen dough, for instance, Japanese Patent Laid-Open No. Hei 11-155559 discloses a baker's yeast having freezing tolerance in a very limited dough having a low sugar concentration, and having drying tolerance. However, there are no cases reported on an yeast capable of exhibiting high freezing tolerance in various doughs from those containing no sugar to those having a high sugar concentration, so that as a matter of course, a dry yeast having such properties has not been reported.

An object of the present invention is to provide an yeast having an excellent leavening ability in various kinds of dough from those containing no sugar to those having a high-sugar concentration, and having drying tolerance, which is suitable for producing bread, especially suitable for producing bread from a frozen dough, specifically an yeast having a high leavening ability in high-sugar content dough to a super-high sugar content dough, and having drying tolerance; an yeast having a high leavening ability in a dough containing no sugar to a high-sugar content dough, and having drying tolerance; an yeast having a high leavening ability in a dough containing no sugar to a low-sugar content dough, and drying tolerance; an yeast having a high freezing tolerance and/or floor resistance in a moderate sugar content dough to a high-sugar content dough, and having drying tolerance; an yeast having a high freezing tolerance and/or floor resistance in a dough containing no sugar to a high-sugar content dough, and having drying tolerance; an yeast having a high freezing tolerance and/or floor resistance in a dough containing no sugar to a low-sugar content dough, and having drying tolerance; and an yeast having low-temperature sensitivity, and having drying tolerance. Another object of the present invention is to provide a dry yeast produced by drying the above-mentioned yeast, which is excellent in preservability and storage stability, and capable of exhibiting a leavening ability to the same extent as that of a raw yeast, especially suitable for producing bread from a frozen dough. Still another object of the present invention is to provide a dough or a frozen dough, comprising the above-mentioned yeast or the above-mentioned dry yeast; and bread having excellent quality stability produced from the above-mentioned dough.

DISCLOSURE OF INVENTION

As a result of intensive studies in view of the above-mentioned problems, the present inventors have found an yeast having the desired properties, and the present invention has been perfected thereby.

Specifically, the present invention relates to:

[1] an yeast having a leavening ability in a high-sugar content dough, and having drying tolerance;

[2] the yeast according to the above item [1], wherein the leavening ability in the form of a dry yeast, as expressed by an amount of gas generated at 30°C for 115 minutes in 85 g of a dough having a sugar concentration of 30% by weight (dry yeast: 1.5% by weight), is 200 ml or more;

[3] the yeast according to the above item [1] or [2], wherein the leavening ability in the form of a dry yeast, as expressed by an amount of gas generated at 30°C for 115 minutes in 85 g of a dough having a sugar concentration of 40% by weight (dry yeast: 1.5% by weight), is 70 ml or more;

[4] an yeast having a leavening ability in a dough having a sugar concentration of 0 to 30% by weight, and having drying tolerance;

[5] the yeast according to the above item [4], wherein the leavening ability in the form of a dry yeast, as expressed by an amount of gas generated at 30°C for 85 minutes in 85 g of a dough having a sugar concentration of 0% by weight (dry yeast: 1% by weight), is 140 ml or more, and wherein the leavening ability in the form of a dry yeast, as expressed by an amount of gas generated at 30°C for 115 minutes in 85 g of a dough having a sugar concentration of 30% by weight (dry yeast: 1.5% by weight), is 200 ml or more;

[6] the yeast according to any one of the above items [1] to [5], wherein the leavening ability in the form of a dry yeast, as expressed by an amount of gas generated at 30°C for 120 minutes in 50 g of a dough after second kneading in the added sugar sponge and dough process (sugar concentration: 28% by weight, dry yeast: 1.5% by weight), is 120 ml or more;

[7] an yeast having a leavening ability in a dough having a sugar concentration of 0 to 5% by weight, and having drying tolerance;

[8] the yeast according to the above item [7], wherein the leavening ability in the form of a dry yeast, as expressed by an amount of gas generated at 30°C for 85 minutes in 85 g of a dough having a sugar concentration of 0% by weight (dry yeast: 1% by weight), is 220 ml or more;

[9] the yeast according to the above item [7] or [8], wherein the leavening ability in the form of a dry yeast, as expressed by an amount of gas generated at 30°C for 85 minutes in 85 g of a dough having a sugar concentration of 5% by weight (dry yeast: 1% by weight), is 160 ml or more;

[10] an yeast having freezing tolerance and/or floor resistance in a dough having a sugar concentration of 10 to 30% by weight, and having drying tolerance;

[11] the yeast according to the above item [10], wherein the leavening ability in the form of a dry yeast, as expressed by an amount of gas generated at 38°C for 120 minutes in 20 g of a dough having a sugar concentration of 10% by weight (dry yeast: 2% by weight), is 90 ml or more in a dough after a 4-week frozen storage following a 60-minute floor time;

[12] the yeast according to the above item [11], wherein the yeast further has a ratio of leavening abilities before and after a 4-week frozen storage following a 60-minute floor time (after frozen storage/before frozen storage) of 0.50 or more;

[13] the yeast according to the above item [11] or [12], wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.20 or more;

[14] the yeast according to the above item [10], wherein the leavening ability in the form of a dry yeast, as expressed by an amount of gas generated at 38°C for 120 minutes in 20 g of a dough having a sugar concentration of 15% by weight (dry yeast: 2.5% by weight), is 70 ml or more in a dough after a 4-week frozen storage following a 60-minute floor time;

[15] the yeast according to the above item [14], wherein the yeast further has a ratio of leavening abilities before and after a 4-week frozen storage following a 60-minute floor time (after frozen storage/before frozen storage) of 0.40 or more;

[16] the yeast according to the above item [14] or [15], wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.20 or more;

[17] the yeast according to the above item [10], wherein the leavening ability in the form of a dry yeast, as expressed by an amount of gas generated at 38°C for 120 minutes in 20 g of a dough having a sugar concentration of 25% by weight (dry yeast: 3% by weight), is 50 ml or more in a dough after a 4-week frozen storage following a 90-minute floor time;

[18] the yeast according to the above item [17], wherein the yeast further has a ratio of leavening abilities before and after a 4-week frozen storage following a 90-minute floor time (after frozen storage/before frozen storage) of 0.60 or more;
[19] the yeast according to the above item [17] or [18], wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.70 or more;
[20] an yeast having freezing tolerance and/or floor resistance in a dough having a sugar concentration of 0 to 30% by weight, and having drying tolerance;
[21] the yeast according to the above item [20], wherein the leavening ability in the form of a dry yeast, as expressed by an amount of gas generated at 38°C for 120 minutes in 20 g of a dough having a sugar concentration of 0% by weight (dry yeast: 2% by weight), is 100 ml or more in a dough after a 4-week frozen storage following a 60-minute floor time;
[22] the yeast according to the above item [21], wherein the yeast further has a ratio of leavening abilities before and after a 4-week frozen storage following a 60-minute floor time (after frozen storage/before frozen storage) of 0.88 or more;
[23] the yeast according to the above item [21] or [22], wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.80 or more;
[24] the yeast according to any one of the above items [20] to [23], wherein the leavening ability in the form of a dry yeast, as expressed by an amount of gas generated at 38°C for 120 minutes in 20 g of a dough having a sugar concentration of 5% by weight (dry yeast: 2% by weight), is 70 ml or more in a dough after a 4-week frozen storage following a 60-minute floor time;
[25] the yeast according to the above item [24], wherein the yeast further has a ratio of leavening abilities before and after a 4-week frozen storage following a 60-minute floor time (after frozen storage/before frozen storage) of 0.40 or more;
[26] the yeast according to the above item [24] or [25], wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.50 or more;
[27] the yeast according to any one of the above items [20] to [26], wherein the leavening ability in the form of a dry yeast, as expressed by an amount of gas generated at 38°C for 120 minutes in 20 g of a dough having a sugar concentration of 10% by weight (dry yeast: 2% by weight), is 90 ml or more in a dough after a 4-week frozen storage following a 60-minute floor time;
[28] the yeast according to the above item [27], wherein the yeast further has a ratio of leavening abilities before and after a 4-week frozen storage following a 60-minute floor time (after frozen storage/before frozen storage) of 0.50 or more;
[29] the yeast according to the above item [27] or [28], wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.20 or more;
[30] the yeast according to any one of the above items [20] to [29], wherein the leavening ability in the form of a dry yeast, as expressed by an amount of gas generated at 38°C for 120 minutes in 20 g of a dough having a sugar concentration of 25% by weight (dry yeast: 3% by weight), is 125 ml or more in a dough after a 4-week frozen storage following a 60-minute floor time, and is 50 ml or more in a dough after a 4-week frozen storage following a 90-minute floor time;
[31] the yeast according to the above item [30], wherein the yeast further has a ratio of leavening abilities before and after a 4-week frozen storage following a 60-minute floor time (after frozen storage/before frozen storage) of 0.70 or more, and a ratio of leavening abilities before and after a 4-week frozen storage following a 90-minute floor time (after frozen storage/before frozen storage) of 0.30 or more;
[32] the yeast according to the above item [30] or [31], wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.35 or more;
[33] an yeast having freezing tolerance and/or floor resistance in a dough having a sugar concentration of 0 to 3% by weight, and having drying tolerance;
[34] the yeast according to the above item [33], wherein the leavening ability in the form of a dry yeast, as expressed by an amount of gas generated at 38°C for 120 minutes in 20 g of a dough having a sugar concentration of 0% by weight (dry yeast: 2% by weight), is 100 ml or more in a dough after a 4-week frozen storage following a 60-minute floor time;
[35] the yeast according to the above item [34], wherein the yeast further has a ratio of leavening abilities before and after a 4-week frozen storage following a 60-minute floor time (after frozen storage/before frozen storage) of 0.88 or more;

[36] the yeast according to the above item [34] or [35], wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.80 or more;

[37] the yeast according to any one of the above items [33] to [36], wherein the leavening ability in the form of a dry yeast, as expressed by an amount of gas generated at 38°C for 120 minutes in 20 g of a dough having a sugar concentration of 3% by weight (dry yeast: 2% by weight), is 50 ml or more in a dough after a 4-week frozen storage following a 60-minute floor time;

[38] the yeast according to the above item [37], wherein the yeast further has a ratio of leavening abilities before and after a 4-week frozen storage following a 60-minute floor time (after frozen storage/before frozen storage) of 0.40 or more;

[39] the yeast according to the above item [37] or [38], wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.35 or more;

[40] an yeast having low-temperature sensitivity, and having drying tolerance;

[41] the yeast according to any one of the above items [1] to [40], wherein the yeast has a remaining leavening ability ratio [ratio of leavening abilities before and after drying (after drying/before drying)] of 0.70 or more;

[42] the yeast according to any one of the above items [1] to [3] and [41], which is *Saccharomyces cerevisiae* D75412 (FERM BP-7688);

[43] the yeast according to any one of the above items [4] to [6] and [41], which is *Saccharomyces cerevisiae* D20946 (FERM BP-7684);

[44] the yeast according to any one of the above items [7] to [9] and [41], which is *Saccharomyces cerevisiae* D46462 (FERM BP-7686);

[45] the yeast according to any one of the above items [10] to [19] and [41], which is *Saccharomyces cerevisiae* D66785 (FERM BP-7687);

[46] the yeast according to any one of the above items [20] to [32] and [41], which is *Saccharomyces cerevisiae* D92764 (FERM BP-7690);

[47] the yeast according to any one of the above items [33] to [39] and [41], which is *Saccharomyces cerevisiae* D80921 (FERM BP-7689);

[48] the yeast according to any one of the above items [40] and [41], which is *Saccharomyces cerevisiae* D31735 (FERM BP-7685);

[49] the yeast according to any one of the above items [1] to [48], wherein the yeast is a dry yeast;

[50] the yeast according to any one of the above items [10] to [49], which is usable for a frozen dough;

[51] a dough comprising the yeast of any one of the above items [1] to [50]; and

[52] bread produced by using the dough of the above item [51].

## BRIEF DESCRIPTION OF THE DRAWINGS

Figure 1 is a graph in which the freezing tolerance of the dry yeast of the present invention is compared with that of a commercially available dry yeast in a dough having a sugar concentration of 0% by weight (subjected to floor time of 60 minutes), wherein solid circles show the results for the inventive dry yeast (D80921), solid squares show those for the inventive dry yeast (D92764), solid triangles show those for a commercially available dry yeast Saf-instant (Red), and open squares show those for a commercially available dry yeast Fermipan Red. In addition, the ordinate shows a leavening ability at each point of frozen storage as the degree of frozen resistance when the leavening ability before frozen storage of each yeast was 1.0. The sugar concentration was shown as 0%, which is the same for each figure hereinbelow.

Figure 2 is a graph in which the freezing tolerance of the dry yeast of the present invention is compared with that of a commercially available dry yeast in a dough having a sugar concentration of 3% by weight (subjected to floor time of 60 minutes), wherein solid circles show the results for the inventive dry yeast (D80921), solid triangles show those for a commercially available dry yeast Saf-instant (Red), and open squares show those for a commercially available dry yeast Fermipan Red. In addition, the ordinate shows a leavening ability at each point of frozen storage as the degree of frozen resistance when the leavening ability before frozen storage of each yeast was 1.0.

Figure 3 is a graph in which the freezing tolerance of the dry yeast of the present invention is compared with that of a commercially available dry yeast in a dough having a sugar concentration of 5% by weight (subjected to floor time of 60 minutes), wherein solid squares show the results for the inventive dry yeast (D92764), solid triangles show those for a commercially available dry yeast Saf-instant (Red), and open squares show those for a commercially available dry yeast Fermipan Red. In addition, the ordinate shows a leavening ability at each point of frozen storage as the degree of frozen resistance when the leavening ability before frozen storage of each yeast was 1.0.

Figure 4 is a graph in which the freezing tolerance of the dry yeast of the present invention is compared with that of a commercially available dry yeast in a dough having a sugar concentration of 10% by weight (subjected to floor time of 60 minutes), wherein solid squares show the results for the inventive dry yeast (D92764), solid circles show those for the inventive dry yeast (D66785), solid triangles show those for a commercially available dry yeast Saf-instant (Red), open squares show those for a commercially available dry yeast Fermipan Red, open circles show those for a commercially available dry yeast Saf-instant (Gold), and open squares show those for a commercially available dry yeast Fermipan Brown. In addition, the ordinate shows a leavening ability at each point of frozen storage as the degree of frozen resistance when the leavening ability before frozen storage of each yeast was 1.0.

Figure 5 is a graph in which the freezing tolerance of the dry yeast of the present invention is compared with that of a commercially available dry yeast in a dough having a sugar concentration of 15% by weight (subjected to floor time of 60 minutes), wherein solid circles show the results for the inventive dry yeast (D66785), open circles show those for a commercially available dry yeast Saf-instant (Gold), and open triangles show those for a commercially available dry yeast Fermipan Brown. In addition, the ordinate shows a leavening ability at each point of frozen storage as the degree of frozen resistance when the leavening ability before frozen storage of each yeast was 1.0.

Figure 6 is a graph in which the freezing tolerance of the dry yeast of the present invention is compared with that of a commercially available dry yeast in a dough having a sugar concentration of 25% by weight (subjected to floor time of 90 minutes), wherein solid circles show the results for the inventive dry yeast (D66785), solid squares show those for the inventive dry yeast (D92764), open circles show those for a commercially available dry yeast Saf-instant (Gold), and open triangles show those for a commercially available dry yeast Fermipan Brown. In addition, the ordinate shows a leavening ability at each point of frozen storage as the degree of frozen resistance when the leavening ability before frozen storage of each yeast was 1.0.

Figure 7 is a graph in which the floor resistance of the dry yeast of the present invention is compared with that of a commercially available dry yeast in a dough having a sugar concentration of 0% by weight, wherein solid squares show the results for the inventive dry yeast (D92764), solid circles show those for the inventive dry yeast (D80921), solid triangles show those for a commercially available dry yeast Saf-instant (Red), and open squares show those for a commercially available dry yeast Fermipan Red. In addition, the ordinate shows a floor resistance as expressed by a ratio of the leavening abilities (60-minute floor time/0-minute floor time) after frozen storage.

Figure 8 is a graph in which the floor resistance of the dry yeast of the present invention is compared with that of a commercially available dry yeast in a dough having a sugar concentration of 3% by weight, wherein solid circles show the results for the inventive dry yeast (D80921), solid triangles show those for a commercially available dry yeast Saf-instant (Red), and open squares show those for a commercially available dry yeast Fermipan Red. In addition, the ordinate shows a floor resistance as expressed by a ratio of the leavening abilities (60-minute floor time/0-minute floor time) after frozen storage.

Figure 9 is a graph in which the floor resistance of the dry yeast of the present invention is compared with that of a commercially available dry yeast in a dough having a sugar concentration of 5% by weight, wherein solid squares show the results for the inventive dry yeast (D92764), solid triangles show those for a commercially available dry yeast Saf-instant (Red), and open squares show those for a commercially available dry yeast Fermipan Red. In addition, the ordinate shows a floor resistance as expressed by a ratio of the leavening abilities (60-minute floor time/0-minute floor time) after frozen storage.

Figure 10 is a graph in which the floor resistance of the dry yeast of the present invention is compared with that of a commercially available dry yeast in a dough having a sugar concentration of 10% by weight, wherein solid squares show the results for the inventive dry yeast (D92764), solid circles show those for the inventive dry yeast (D66785), solid triangles show those for a commercially available dry yeast Saf-instant (Red), open circles show those for a commercially available dry yeast Saf-instant (Gold), open squares show those for a commercially available dry yeast Fermipan Red, and open triangles show those for a commercially available dry yeast Fermipan Brown. In addition, the ordinate shows a floor resistance as expressed by a ratio of the leavening abilities (90-minute floor time/30-minute floor time) after frozen storage.

Figure 11 is a graph in which the floor resistance of the dry yeast of the present invention is compared with that of a commercially available dry yeast in a dough having a sugar concentration of 15% by weight, wherein solid circles show the results for the inventive dry yeast (D66785), solid triangles show those for a commercially available dry yeast Saf-instant (Gold), and open squares show those for a commercially available dry yeast Fermipan Brown. In addition, the ordinate shows a floor resistance as expressed by a ratio of the leavening abilities (90-minute floor time/30-minute floor time) after frozen storage.

Figure 12 is a graph in which the floor resistance of the dry yeast of the present invention is compared with that of a commercially available dry yeast in a dough having a sugar concentration of 25% by weight, wherein solid circles show the results for the inventive dry yeast (D66785), solid squares show those for the inventive dry yeast (D92764), open triangles show those for a commercially available dry yeast Fermipan Brown, and open circles show those for a commercially available dry yeast Saf-instant (Gold). In addition, the ordinate shows a floor resistance as expressed by a ratio of the leavening abilities (90-minute floor time/30-minute floor time) after frozen storage.

## BEST MODE FOR CARRYING OUT THE INVENTION

The yeast of the present invention has an excellent leavening ability in various doughs from those containing no sugar to those having a high sugar concentration, and drying tolerance. In addition, the yeast has especially excellent properties for producing bread from a dough having a specified sugar concentration range, specifically including excellent leavening ability, freezing tolerance, floor resistance and low-temperature sensitivity.

According to the yeast of the present invention, since the yeast is not necessarily used selectively depending upon the sugar concentration of bread as in conventional yeasts, the yeast can meet the need for producing bread having all sorts of general sugar concentrations. In addition, bread having a specified sugar concentration is produced by using the yeast capable of exhibiting especially excellent properties at the specified sugar concentration, whereby more excellent bread can be produced. In addition, the dry yeast produced by drying the yeast has excellent preservability and storage stability, and can exhibit a leavening ability to the same extent as that of the raw yeast in a dough having a high sugar content to a super-high sugar content, or a dough having a wide range of sugar concentrations of from a dough containing no sugar to a high-sugar content dough, or a dough containing no sugar to a dough having a low-sugar content, so that high quality bread can be generally produced in all sorts of sugar concentration ranges. Also, there are no conventional dry yeasts which have, for instance, satisfactory freezing tolerance and/or floor resistance, so that it was difficult to use the conventional dry yeast in the frozen dough process. By contrast, the above-mentioned dry yeast can exhibit high freezing tolerance and/or floor resistance in a dough having a moderate-sugar content to a high-sugar content, or a dough having a wide range of sugar concentrations of from a dough containing no sugar to a high-sugar content dough, or a dough containing no sugar to a dough having a low-sugar content, and having drying tolerance. Therefore, the yeast can be very favorably used in the frozen dough process without being substantially limited by a sugar concentration.

Incidentally, the dry yeast can be roughly classified into two kinds according to its producing process and properties. One is a generally so-called "active dry yeast" having a cell moisture content of about 10% by weight which does not require a specialized equipment in its production. Upon use, the active dry yeast is dissolved in warm water (in some cases sucrose is added thereto) to activate the dry yeast over a period of several dozen minutes, and thereafter the activated yeast is kneaded into a bread dough. The other is a so-called instant dry yeast having a cell moisture content of about 4% by weight, so that the yeast can be stored for a long period of time. Upon use, the instant dry yeast can be directly kneaded into a dough without being activated in warm water.

The term "dry yeast" as referred to herein is an instant dry yeast, which is an yeast having more excellent drying tolerance as compared to those yeasts which can only be used as the above-mentioned active dry yeasts. Therefore, the dry yeast of the present invention has very excellent preservability, and can be directly added to a dough during kneading without activation with warm water.

Furthermore, according to the present invention, there are obtained, for instance, a dough produced by a scratch process and a dough suitable for a frozen dough process, comprising the above-mentioned yeast or the above-mentioned dry yeast, so that there can be provided excellent bread having stable quality with the dough.

Here, the above-mentioned dry yeast, dough and bread are encompassed in the present invention. In the present specification, when "% by weight" is used as a sugar concentration of the dough, it means "parts by weight of the sugar based on 100 parts by weight of flour" in accordance with the ordinary practice in the field of art. For instance, when it is stated "a dough having a sugar concentration of 5% by weight," it is referred to "a dough produced by adding 5 parts by weight of the sugar to 100 parts by weight of flour." The frozen dough may include the concept of the refrigerated dough in some cases. Also, the term "dough containing no sugar" as referred to herein refers to a dough having a sugar concentration of 0% by weight, the term "low-sugar content dough" refers to a dough having a sugar concentration of exceeding 0% by weight to 10% by weight, the term "moderate-sugar content dough" refers to a dough having a sugar concentration of exceeding 10% by weight to 15% by weight, the term "high-sugar content dough" refers to a dough having a sugar concentration of exceeding 15% by weight to 30% by weight, and the term "super-high-sugar content dough" refers to a dough having a sugar concentration of exceeding 30% by weight to 40% by weight, respectively. The term "sugar" as referred to herein is generally referred to as sucrose, but the kinds of the sugar are not particularly limited thereto, and any of those sugars may be used, as long as they are usable by addition during the production of the dough. Also, the high-sugar content dough may encompass the meaning of the super-high-sugar content dough in some cases.

The properties of the yeast of the present invention will be explained hereinbelow.

Each of the doughs having a sugar concentration of 0% by weight, a sugar concentration of 5% by weight, a sugar concentration of 30% by weight, and a sugar concentration of 40% by weight, produced according to the scratch production process is produced by kneading each of the raw materials in accordance with the composition shown in Table 1 at a temperature after termination of kneading of 29°C with a table mixer (manufactured by HOBART).

Table 1

| Composition of Scratch Dough | | | | |
|---|---|---|---|---|
| | Dough Having Sugar Conc. of 0% by weight | Dough Having Sugar Conc. of 5% by weight | Dough Having Sugar Conc. of 30% by weight | Dough Having Sugar Conc. of 40% by weight |
| Flour | 100 g | 100 g | 100 g | 100 g |
| Sucrose | 0 g | 5 g | 30 g | 40 g |
| Salt | 0.5 g | 2.0 g | 0.5 g | 0.5 g |
| Dry Yeast | 1.0 g | 1.0 g | 1.5 g | 1.5 g |
| Water | 65 ml | 62 ml | 52 ml | 47 ml |

Similarly, each of the doughs having a sugar concentration of 0% by weight, a sugar concentration of 3% by weight, a sugar concentration of 5% by weight, a sugar concentration of 10% by weight, a sugar concentration of 15% by weight, and a sugar concentration of 25% by weight, produced according to the frozen dough production process is produced by kneading each of the raw materials in accordance with the composition shown in Table 4 mentioned later at a temperature after termination of kneading of 29°C with a table mixer (manufactured by HOBART).
The definition for each of the properties of the yeast of the present invention and the evaluation methods thereof are collectively given.

(1) Leavening Ability in Dough Having Sugar Concentration of 0% by Weight

The leavening ability in a dough having a sugar concentration of 0% by weight as referred to herein is expressed by an amount of gas (carbon dioxide gas) generated in a dough as determined under given conditions, wherein the dough is produced by using a dry yeast obtained by drying an yeast as mentioned below. Concretely, the amount of gas generated (ml) is obtained by kneading ingredients in accordance with the composition of a dough having a sugar concentration of 0% by weight, to give a dough, dividing the dough into a 85 g-portion, and determining the amount of gas generated by Fermograph (manufactured by ATTO Co., LTD., Japan) at 30°C for 85 minutes according to a conventional process.

(2) Leavening Ability in Dough Having Sugar Concentration of 5% by weight

The leavening ability in a dough having a sugar concentration of 5% by weight as referred to herein is expressed by an amount of gas generated in a dough as determined under given conditions, wherein the dough is produced by using a dry yeast obtained by drying an yeast as mentioned below. Concretely, the amount of gas generated (ml) is obtained by kneading ingredients in accordance with the composition of a dough having a sugar concentration of 5% by weight, to give a dough, dividing the dough into a 85 g-portion, and determining the amount of gas generated by Fermograph (manufactured by ATTO Co., LTD., Japan) at 30°C for 85 minutes according to a conventional process.

(3) Leavening Ability in Dough Having Sugar Concentration of 30% by weight

The leavening ability in a dough having a sugar concentration of 30% by weight as referred to herein is expressed by an amount of gas generated in a dough as determined under given conditions, wherein the dough is produced by using a dry yeast obtained by drying an yeast as mentioned below. Concretely, the amount of gas generated (ml) is obtained by kneading ingredients in accordance with the composition of a dough having a sugar concentration of 30% by weight, to give a dough, dividing the dough into a 85 g-portion, and determining the amount of gas generated by Fermograph (manufactured by ATTO Co., LTD., Japan) at 30°C for 115 minutes according to a conventional process.

(4) Leavening Ability in Dough Having Sugar Concentration of 40% by weight

The leavening ability in a dough having a sugar concentration of 40% by weight as referred to herein is expressed by an amount of gas generated in a dough as determined under given conditions, wherein the dough is produced by using a dry yeast obtained by drying an yeast as mentioned below. Concretely, the amount of gas generated (ml) is obtained by kneading ingredients in accordance with the composition of a dough having a sugar concentration of 40% by weight, to give a dough, dividing the dough into a 85 g-portion, and determining the amount of gas generated by Fermograph (manufactured by ATTO Co., LTD., Japan) at 30°C for 115 minutes according to a conventional process.

(5) Amount of Gas Generated in Dough After Second Kneading (*Hongone*)

The amount of gas generated in a dough after second kneading as referred to herein is expressed by an amount of gas generated in a dough after second kneading as determined under given conditions, wherein the dough is produced according to the added sugar sponge and dough process by using a dry yeast obtained by drying an yeast as mentioned below. Concretely, the amount of gas generated (ml) is obtained by producing a dough in accordance with the composition for sponge and dough produced by the added sugar sponge and dough process shown in Table 2 under the conditions for producing a sponge and dough in the added sugar sponge and dough process shown in Table 3, dividing the dough after second kneading into a 50 g-portion, and determining the amount of gas generated by Fermograph (manufactured by ATTO Co., LTD., Japan) at 30°C for 120 minutes according to a conventional process.

Table 2

| Composition for Sponge and Dough Produced by Added Sugar Sponge and Dough Process | | |
|---|---|---|
| | Sponge | Dough |
| Flour | 70 parts by wt. | 30 parts by wt. |
| Sugar | 3 | 25 |
| Salt | - | 1 |
| Fats and Oils | - | 8 |
| Yeast | 1.5 | - |
| Yeast Food | 0.1 | - |
| Emulsifier | 0.3 | - |
| Defatted Powder | - | 2 |
| Whole Egg | - | 8 |
| Water | 40 | 13 |

Table 3

| Conditions for Producing Sponge and Dough in Added Sugar Sponge and Dough Process | | |
|---|---|---|
| | Sponge | Dough |
| Mixing Conditions | L3M2 | L2M7, followed by adding fats and oils L2H4 |
| Temperature After Termination of Kneading | 26°C | 27°C |
| Leavening Time | 28°C, 2h. 30 min. | |
| Floor Time | | 1 h |
| Divided Amount | | 330 g |
| Bench Time | | 25 min. |
| Molding | | roll-shape |
| Final Proof (*Hoiro*) | | 38°C, 55 min. |
| Baking | | 200°C, 20 min. |

(6) Freezing Tolerance

The freezing tolerance as referred to herein is a property in which a dry yeast in a dough after frozen storage for a given period of time can exhibit a leavening ability sufficient for use to the same extent as that of the dry yeast before frozen storage, wherein the dry yeast is produced by drying an yeast as mentioned below, and wherein the dough is produced using the dry yeast. Here, the leavening ability after frozen storage is expressed as an amount of gas generated (ml) in a dough obtained by dividing a dough obtained in accordance with the composition of Table 4 into a 20 g-portion, allowing the dough to take a floor time of 60 or 90 minutes, subjecting the dough to frozen storage at -20°C for 4 weeks, thawing the dough at 25°C for 30 minutes, and determining the amount of gas generated in the dough by Fermograph (manufactured by ATTO Co., LTD., Japan) at 38°C for 120 minutes.

Table 4

| Composition Table for Frozen Dough | | | | | | |
|---|---|---|---|---|---|---|
| | Dough Having Sugar Conc. of 0% by weight | Dough Having Sugar Conc. of 3% by weight | Dough Having Sugar Conc. of 5% by weight | Dough Having Sugar Conc. of 10% by weight | Dough Having Sugar Conc. of 15% by weight | Dough Having Sugar Conc. of 25% by weight |
| Flour | 100 g | 100 g | 100 g | 100 g | 100 g | 100 g |
| Sucrose | 0 g | 3 g | 5 g | 10 g | 15 g | 25 g |
| Salt | 0.5 g | 0.5 g | 0.5 g | 0.5 g | 0.5 g | 0.5 g |
| Dry Yeast | 2 g | 2 g | 2 g | 2 g | 2.5 g | 3 g |
| Water | 65 ml | 64 ml | 63 ml | 60 ml | 58 ml | 54 ml |

In addition, the freezing tolerance can be expressed as a ratio of the leavening abilities before and after frozen storage (after frozen storage/before frozen storage), specifically a ratio of the amounts of gas generated expressing the leavening ability before and after frozen storage, which is suitable for evaluating freezing tolerance from the viewpoint such that the extent of the leavening ability after frozen storage as compared to that before frozen storage can be directly evaluated. The yeast of the present invention is more preferably those in which both the leavening ability after frozen storage and the ratio of the leavening abilities before and after frozen storage mentioned above are high. The leavening ability before frozen storage is expressed as the amount of gas generated in the dough when a dough is not subjected to frozen storage in the above-mentioned method for the leavening ability after frozen storage.

(7) Floor Resistance

The floor resistance as referred to herein is a property in which a dry yeast in a dough can exhibit a leavening ability sufficient for use to the same extent as that of the dry yeast in the dough after frozen storage with no or substantially no pre-leavening (floor) even if the pre-leavening is carried out before frozen storage, wherein the dry yeast is produced by drying an yeast as mentioned below, and wherein the dough is produced using the dry yeast. The floor resistance is expressed as a ratio of the leavening ability in the case where a dough is subjected to a long floor time to the leavening ability in the case where a dough is subjected to a short floor time (long floor time/short floor time), which is obtained by producing doughs by using the above-mentioned dry yeast, subjecting each of doughs to a short floor time or a long floor time, and obtaining the ratio after subjecting the dough to frozen storage for a given period of time, wherein the ratio is specifically obtained as a ratio of the amounts of gas generated in the above two cases obtained as indices of leavening ability in the same manner as the freezing tolerance.

Specifically, a dough having a sugar concentration of 0% by weight, a dough having a sugar concentration of 3% by weight, or a dough having a sugar concentration of 5% by weight is subjected to frozen storage for 4 weeks following a floor time of 0 minutes or 60 minutes, and thereafter thawed, and a leavening ability is obtained as an amount of gas generated in each case, and a ratio of leavening abilities is taken. Also, a dough having a sugar concentration of 10% by weight, a dough having a sugar concentration of 15% by weight or a dough having a sugar concentration of 25% by weight is subjected to frozen storage for 4 weeks following a floor time of 30 minutes or 90 minutes and thereafter thawed, and a leavening ability is obtained as an amount of gas generated in each case, and a ratio of leavening abilities is taken. Here, the amount of gas generated (ml) is obtained by producing a dough in accordance with the composition shown in Table 4 by using the dry yeast obtained by drying an yeast as mentioned below, dividing the dough into a 20 g-portion, allowing the dough to take a given floor time at 30°C, subjecting the dough to frozen storage at -20°C for 4 weeks, followed by thawing the dough at 25°C for 30 minutes, and determining the amount of gas generated in the dough by Fermograph (manufactured by ATTO Co., LTD., Japan) at 38°C for 120 minutes.

(8) Low-Temperature Sensitivity

The low-temperature sensitivity as referred to herein is a property showing a low leavening ability at a low temperature of preferably from 0° to 10°C, more preferably from 3° to 8°C. Concretely, in the present invention, the low-temperature sensitivity is evaluated by a ratio of the leavening ability in a dough at 30°C of the dry yeast obtained by drying an yeast mentioned later to a ratio of the leavening ability at 5°C of the dry yeast (leavening ability at 30°C/leavening ability at 5°C). The larger the value for the ratio, the more excellent the low-temperature sensitivity.

Since the leavening ability at 5°C of the dry yeast is very small, the method for determining the leavening ability differs between the leavening ability at 5°C and the leavening ability at 30°C. Specifically, when the leavening ability at 5°C was determined, each of the ingredients in a dough composition shown in Table 5 is mixed and kneaded with a table mixer so as to have a temperature after termination of kneading of 25°C to give a dough, and a cylinder is charged with the resulting dough to previously determine its initial volume (ml). Next, the dough is allowed to leaven in this state at 5°C for 20 hours, and thereafter the volume of the dough (volume after leavening) was determined. The difference of the volume after leavening and the initial volume (volume after leavening - initial volume) is defined as the leavening ability at 5°C. On the other hand, as to the leavening ability at 30°C, each of the ingredients in a dough composition shown in Table 5 is mixed and kneaded so as to have a temperature after termination of kneading of 29°C in the same manner to give a dough, and the resulting dough is divided into 85-g portions, and the amount of gas generated in the dough is determined by Fermograph (manufactured by ATTO Co., LTD., Japan) at 30°C for 85 minutes according to a conventional process. The amount of gas generated is defined as the leavening ability at 30°C. Here, when a raw yeast (compressed yeast) is used as an yeast, 2 g of a raw yeast is used in place of 1 g of the dry yeast in Table 5.

Table 5

| Dough Composition for Determining Leavening Ability at 5°C and That for Determining Leavening Ability at 30°C | | |
|---|---|---|
| | Leavening Ability of Compressed Yeast | Leavening Ability of Dry Yeast |
| Flour | 100 g | 100 g |
| Sucrose | 5 g | 5 g |
| Salt | 2 g | 2 g |
| Dry Yeast | 0 g | 1 g |
| Compressed Yeast | 2 g | 0 g |
| Water | 62 ml | 62 ml |

(9) Drying Tolerance

The drying tolerance in the present invention is expressed as a ratio of the leavening ability of the yeast after drying to the leavening ability of the yeast before drying (remaining leavening ability ratio). The yeast of the present invention is excellent in drying tolerance. Accordingly, the yeast even after drying can exhibit a leavening ability sufficient for use to the same extent as that of the yeast before drying.

Here, the remaining leavening ability ratio is obtained as follows. Specifically, each of the ingredients is mixed and kneaded (temperature after termination of kneading: 29°C) in accordance with the composition of Table 6 with a table mixer (manufactured by HOBERT) using each yeast before and after drying to give a dough. The resulting dough is divided into 85-g portions, and thereafter the amount of gas generated (ml) is determined by Fermograph at 30°C for 85 minutes for each case. The amount of gas generated is defined as the leavening ability. Next, the remaining leavening ability ratio is obtained by using the leavening ability of the yeast after drying, the leavening ability of the yeast before drying, the water content (% by weight) of cell after drying and the water content (% by weight) of cell before drying according to the following equation:

Remaining Leavening Ability Ratio = [Leavening Ability of Yeast After Drying/(100 - Cell Moisture Content After Drying)]/[Leavening Ability of Yeast Before Drying/(100 - Cell Moisture Content Before Drying)] .

The moisture contents of cell after drying and before drying are determined as follows. About 1 g of cells to be determined are accurately weighed (Cell Weight 1; g), and the cells are dried at 110°C for 12 hours in a sufficiently dried test tube, and thereafter the dried cells are accurately weighed again (Cell Weight 2; g). The water content is obtained by the following equation:

Cell Water Content (% by weight) = [(Cell Weight 1 - Cell Weight 2)/Cell Weight 1] × 100.

Table 6

| Dough Composition for Determining Remaining Leavening Ability Ratio | | |
|---|---|---|
| | Leavening Ability Before Drying | Leavening Ability After Drying |
| Flour | 100 g | 100 g |
| Sucrose | 5 g | 5 g |
| Salt | 2 g | 2 g |
| Dry Yeast | 0 g | 1 g |
| Compressed Yeast | 2 g | 0 g |
| Water | 62ml | 62ml |

In one embodiment of the present invention, there is provided an yeast which is especially suitably used for a high-sugar content dough, having a high leavening ability in the dough, and having drying tolerance. As the yeast, those having the following properties are preferred, and can sufficiently exhibit the desired effects of the present invention.

The leavening ability in a dough having a sugar concentration of 30% by weight is preferably 200 ml or more, more preferably 250 ml or more, and/or the leavening ability in a dough having a sugar concentration of 40% by weight is preferably 70 ml or more, more preferably 90 ml or more. In addition, it is more preferable that the amount of gas generated in a dough after second kneading is preferably 120 ml or more, more preferably 170 ml or more, especially preferably 175 ml or more, still more preferably 190 ml or more.

As the drying tolerance, the remaining leavening ability ratio is preferably 0.70 or more, more preferably 0.80 or more. In this embodiment, when the remaining leavening ability ratio is 0.70 or more, the yeast can be suitably used as an yeast for frozen dough.

Concrete one example of the preferred yeast in this embodiment includes *Saccharomyces cerevisiae* D75412 (FERM BP-7688).

In another embodiment of the present invention, there is provided an yeast which is suitably used for a dough having a sugar concentration of from 0 to 30% by weight, having a high leavening ability in the dough, and having drying tolerance. As the yeast, those having the following properties are preferred, and can sufficiently exhibit the desired effects of the present invention.

The leavening ability in a dough having a sugar concentration of 0% by weight is preferably 140 ml or more, more preferably 190 ml or more, and the leavening ability in a dough having a sugar concentration of 30% by weight is preferably 200 ml or more, more preferably 230 ml or more. In addition, it is more preferable that the amount of gas generated in a dough after second kneading is preferably 120 ml or more, more preferably 170 ml or more, especially preferably 175 ml or more.

As the drying tolerance, the remaining leavening ability ratio is preferably 0.70 or more, more preferably 0.80 or more. In this embodiment, when the remaining leavening ability ratio is 0.70 or more, the yeast can be suitably used as an yeast for frozen dough.

Concrete one example of the preferred yeast in this embodiment includes *Saccharomyces cerevisiae* D20946 (FERM BP-7684).

In another embodiment of the present invention, there is provided an yeast which is suitably used for a dough having a sugar concentration of from 0 to 5% by weight, having a high leavening ability in the dough, and having drying tolerance. As the yeast, those having the following properties are preferred, and can sufficiently exhibit the desired effects of the present invention.

The leavening ability in a dough having a sugar concentration of 0% by weight is preferably 220 ml or more, more preferably 240 ml or more, and/or the leavening ability in a dough having a sugar concentration of 5% by weight is preferably 160 ml or more, more preferably 180 ml or more.

As the drying tolerance, the remaining leavening ability ratio is preferably 0.70 or more, more preferably 0.80 or more. In this embodiment, when the remaining leavening ability ratio is 0.70 or more, the yeast can be suitably used as an yeast for frozen dough.

Concrete one example of the preferred yeast in this embodiment includes *Saccharomyces cerevisiae* D46462 (FERM BP-7686).

In another embodiment of the present invention, there is provided an yeast which is suitably used for a dough having a sugar concentration of from 10 to 30% by weight, having a freezing tolerance and/or floor resistance in the dough, and having drying tolerance. As the yeast, those having the following properties are preferred, and can sufficiently exhibit the desired effects of the present invention.

When a dough has a sugar concentration of 10% by weight, it is preferable that the leavening ability in the dough after a 4-week frozen storage following a 60-minute floor time, as expressed by an amount of gas generated, is preferably 90 ml or more, more preferably 100 ml or more, and further that the ratio of the leavening abilities before and after a 4-week frozen storage following a 60-minute floor time is preferably 0.50 or more, more preferably 0.55 or more. In addition, it is more preferable that the ratio of the leavening ability after a 4-week frozen storage following a 90-minute floor time to the leavening ability after a 4-week frozen storage following a 30-minute floor time (90-minute floor time/30-minute floor time) is preferably 0.20 or more, more preferably 0.35 or more.

When a dough has a sugar concentration of 15% by weight, it is preferable that the leavening ability in the dough after a 4-week frozen storage following a 60-minute floor time, as expressed by an amount of gas generated, is preferably 70 ml or more, more preferably 100 ml or more, still more preferably 130 ml or more, and further that the ratio of the leavening abilities before and after a 4-week frozen storage following a 60-minute floor time is preferably 0.40 or more, more preferably 0.50 or more, still more preferably 0.65 or more. In addition, it is more preferable that the ratio of the leavening ability after a 4-week frozen storage following a 90-minute floor time to the leavening ability after a 4-week frozen storage following a 30-minute floor time (90-minute floor time/30-minute floor time) is preferably 0.20 or more, more preferably 0.30 or more, still more preferably 0.45 or more.

When a dough has a sugar concentration of 25% by weight, it is preferable that the leavening ability in the dough after a 4-week frozen storage following a 90-minute floor time, as expressed by an amount of gas generated, is preferably 50 ml or more, more preferably 60 ml or more, still more preferably 90 ml or more, and further that the ratio of the leavening abilities before and after a 4-week frozen storage following a 90-minute floor time is preferably 0.60 or more, more preferably 0.63 or more. In addition, it is more preferable that the ratio of the leavening ability after a 4-week frozen storage following a 90-minute floor time to the leavening ability after a 4-week frozen storage following a 30-minute floor time (90-minute floor time/30-minute floor time) is preferably 0.70 or more, more preferably 0.78 or more.

As the drying tolerance, the remaining leavening ability ratio is preferably 0.70 or more, more preferably 0.80 or more.

Concrete one example of the preferred yeast in this embodiment includes *Saccharomyces cerevisiae* D66785 (FERM BP-7687).

In another embodiment of the present invention, there is provided an yeast which is suitably used for a dough having a sugar concentration of from 0 to 30% by weight, having freezing tolerance and/or floor resistance in the dough, and having drying tolerance. As the yeast, those having the following properties are preferred, and can sufficiently exhibit the desired effects of the present invention.

When a dough has a sugar concentration of 0% by weight, it is preferable that the leavening ability in the dough after a 4-week frozen storage following a 60-minute floor time, as expressed by an amount of gas generated, is preferably 100 ml or more, more preferably 105 ml or more, and further that the ratio of the leavening abilities before and after a 4-week frozen storage following a 60-minute floor time is preferably 0.88 or more, more preferably 0.90 or more. In addition, it is more preferable that the ratio of the leavening ability after a 4-week frozen storage following a 60-minute floor time to the leavening ability after a 4-week frozen storage following a 0-minute floor time (60-minute floor time/0-minute floor time) is preferably 0.80 or more, more preferably 0.90 or more.

Further, when a dough has a sugar concentration of 5% by weight, it is preferable that the leavening ability in the dough after a 4-week frozen storage following a 60-minute floor time, as expressed by an amount of gas generated, is preferably 70 ml or more, more preferably 85 ml or more, still more preferably 90 ml or more, and further that the ratio of the leavening abilities before and after a 4-week frozen storage following a 60-minute floor time is preferably 0.40 or more, more preferably 0.70 or more. In addition, it is more preferable that the ratio of the leavening ability after a 4-week frozen storage following a 60-minute floor time to the leavening ability after a 4-week frozen storage following a 0-minute floor time (60-minute floor time/0-minute floor time) is preferably 0.50 or more, more preferably 0.60 or more.

Further, when a dough has a sugar concentration of 10% by weight, it is preferable that the leavening ability in the dough after a 4-week frozen storage following a 60-minute floor time, as expressed by an amount of gas generated, is preferably 90 ml or more, more preferably 100 ml or more, and further that the ratio of the leavening abilities before and after a 4-week frozen storage following a 60-minute floor time is preferably 0.50 or more, more preferably 0.55 or more, still more preferably 0.65 or more. In addition, it is more preferable that the ratio of the leavening ability after a 4-week frozen storage following a 90-minute floor time to the leavening ability after a 4-week frozen storage following a 30-minute floor time (90-minute floor time/30-minute floor time) is preferably 0.20 or more, more preferably 0.35 or more, still more preferably 0.40 or more.

Further, when a dough has a sugar concentration of 25% by weight, it is preferable that the leavening ability in the dough after a 4-week frozen storage following a 60-minute floor time, as expressed by an amount of gas generated, is preferably 125 ml or more, more preferably 130 ml or more, still more preferably 140 ml or more, and the leavening ability in the dough after a 4-week frozen storage following a 90-minute floor time, as expressed by an amount of gas generated, is preferably 50 ml or more, more preferably 60 ml or more, still more preferably 90 ml or more.

Further, it is preferable that the ratio of the leavening abilities before and after a 4-week frozen storage following a 60-minute floor time is preferably 0.70 or more, more preferably 0.72 or more, and that the ratio of the leavening abilities before and after a 4-week frozen storage following a 90-minute floor time is preferably 0.30 or more, more preferably 0.35 or more. In addition, it is more preferable that the ratio of the leavening ability after a 4-week frozen storage following a 90-minute floor time to the leavening ability after a 4-week frozen storage following a 30-minute floor time (90-minute floor time/30-minute floor time) is preferably 0.35 or more, more preferably 0.38 or more.

As the drying tolerance, the remaining leavening ability ratio is preferably 0.70 or more, more preferably 0.80 or more.

Concrete one example of the preferred yeast in this embodiment includes *Saccharomyces cerevisiae* D92764 (FERM BP-7690).

In another embodiment of the present invention, there is provided an yeast which is suitably used for a dough having a sugar concentration of from 0 to 3% by weight, having freezing tolerance and/or floor resistance in the dough, and having drying tolerance. As the yeast, those having the following properties are preferred, and can sufficiently exhibit the desired effects of the present invention.

When a dough has a sugar concentration of 0% by weight, it is preferable that the leavening ability in the dough after a 4-week frozen storage following a 60-minute floor time, as expressed by an amount of gas generated, is preferably 100 ml or more, more preferably 110 ml or more, and further that the ratio of the leavening abilities before and after a 4-week frozen storage following a 60-minute floor time is preferably 0.88 or more, more preferably 0.95 or more. In addition, it is more preferable that the ratio of the leavening ability after a 4-week frozen storage following a 60-minute floor time to the leavening ability after a 4-week frozen storage following a 0-minute floor time (60-minute floor time/0-minute floor time) is preferably 0.80 or more, more preferably 0.90 or more.

Further, when a dough has a sugar concentration of 3% by weight, it is preferable that the leavening ability in the dough after a 4-week frozen storage following a 60-minute floor time, as expressed by an amount of gas generated, is preferably 50 ml or more, more preferably 70 ml or more, still more preferably 75 ml or more, and further that the ratio of the leavening abilities before and after a 4-week frozen storage following a 60-minute floor time is preferably 0.40 or more, more preferably 0.50 or more, still more preferably 0.55 or more. In addition, it is more preferable that the ratio of the leavening ability after a 4-week frozen storage following a 60-minute floor time to the leavening ability after a 4-week frozen storage following a 0-minute floor time (60-minute floor time/0-minute floor time) is preferably 0.35 or more, more preferably 0.50 or more.

As the drying tolerance, the remaining leavening ability ratio is preferably 0.70 or more, more preferably 0.80 or more.

Concrete one example of the preferred yeast in this embodiment includes *Saccharomyces cerevisiae* D80921 (FERM BP-7689).

In another embodiment of the present invention, there is provided an yeast which is especially suitably used for a low-sugar content dough, having low-temperature sensitivity in the dough, and having drying tolerance. As to the low-temperature sensitivity, the ratio of the leavening ability in the dough at 30°C to the leavening ability in the dough at 5°C (leavening ability in the dough at 30°C/ leavening ability in the dough at 5°C) is preferably 0.70 or more, more preferably 0.80 or more. On the other hand, as the drying tolerance, the remaining leavening ability ratio is preferably 0.70 or more, more preferably 0.80 or more.

Concrete one example of the preferred yeast in this embodiment includes *Saccharomyces cerevisiae* D31735 (FERM BP-7685).

The yeast of the present invention includes all strains having the properties as described above in a dough containing no sugar to a dough having a high sugar concentration, especially including all strains exhibiting a high leavening ability in a dough having a high-sugar content to a super-high sugar content, and having drying tolerance; all strains exhibiting a high leavening ability in a dough containing no sugar to a dough having a high sugar content, and having drying tolerance; all strains exhibiting a high leavening ability in a dough containing no sugar to a dough having a low sugar content, and having drying tolerance; all strains having a high freezing tolerance and/or floor resistance in a dough having a moderate-sugar content to a high-sugar content, and having drying tolerance; all strains having a high freezing tolerance and/or floor resistance in a dough containing no sugar to a dough having a high-sugar content, and having drying tolerance; all strains having a high freezing tolerance and/or floor resistance in a dough containing no sugar to a dough having a low-sugar content, and having drying tolerance; all strains having a low-temperature sensitivity, and having drying tolerance, which encompass all of those regardless of being artificially produced or being isolated from nature. For instance, any of known yeasts may be used, including those yeasts generally utilized in the production of bread such as widely used *Saccharomyces cerevisiae* and other *Saccharomyces uvarium, Saccharomyces exiguus*, and those belonging to the genus of *Torulaspora*, and any of these can be used as long as the desired effects of the present invention can be exhibited.

The yeast of the present invention can be obtained by various known methods. The desired yeast can be obtained by, for instance, carrying out screening widely from nature on the bases of the various properties as mentioned above, and selecting an yeast having the desired properties; or combining each yeast with a known hybridization method or causing various mutations in an yeast according to a known method, and selecting an yeast having the desired properties.

In the present invention, as a result of screening a strain isolated from nature or a grown strain produced by hybridization on the basis of various properties mentioned above, seven novel strains having excellent properties as mentioned above are obtained, each of which was named *Saccharomyces cerevisiae* D75412, *Saccharomyces cerevisiae* D20946, *Saccharomyces cerevisiae* D46462, *Saccharomyces cerevisiae* D66785, *Saccharomyces cerevisiae* D92764, *Saccharomyces cerevisiae* D80921 and *Saccharomyces cerevisiae* D31735, and deposited to The International Patent Organism Depositary, National Institute of Advanced Industrial Science and Technology.

The novel strains will be explained below.

[Mycological Properties]

The mycological properties of the yeast of the present invention are shown in Tables 7 to 20.

Table 7

| Mycological Properties (Part 1) of Inventive Strain D75412 | |
|---|---|
| Morphology of Vegetative Cell | Egg-shape to Oblong-shape (3-8) × (6-10) μm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |
| Growth in Vitamin-Lacking Medium | Not growing |
| Growth in the Presence of Cycloheximide (1000 ppm) (100 ppm) | Not growing Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 8

| Mycological Properties (Part 2) of Inventive Strain D75412 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | + | + |
| Galactose | + | + |
| Sucrose | + | + |
| Maltose | + | + |
| Lactose | - | - |
| Raffinose | + | + |
| Cellobiose | - | N.T. |
| Trehalose | + | - |

Table 9

| Mycological Properties (Part 1) of Inventive Strain D20946 | |
|---|---|
| Morphology of Vegetative Cell | Egg-shape to Oblong-shape (3-8) × (6-10) µm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |
| Growth in Vitamin-Lacking Medium | Not growing |
| Growth in the Presence of Cycloheximide (1000 ppm) (100 ppm) | Not growing Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 10

| Mycological Properties (Part 2) of Inventive Strain D20946 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | N.T. | + |
| Galactose | + | + |
| Sucrose | + | + |
| Maltose | + | + |
| Lactose | - | - |
| Raffinose | N.T. | - |
| Cellobiose | - | N.T. |
| Trehalose | + | N.T. |

Table 11

| Mycological Properties (Part 1) of Inventive Strain D46462 | |
|---|---|
| Morphology of Vegetative Cell | Egg-shape to Oblong-shape (3-6) × (4-10) µm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |

Table 11 (continued)

| Mycological Properties (Part 1) of Inventive Strain D46462 | |
|---|---|
| Growth in Vitamin-Lacking Medium | Not growing |
| Growth in the Presence of Cycloheximide (1000 ppm)<br>(100 ppm) | Not growing<br>Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 12

| Mycological Properties (Part 2) of Inventive Strain D46462 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | + | + |
| Galactose | + | + |
| Sucrose | + | + |
| Maltose | + | + |
| Lactose | - | - |
| Raffinose | + | N.T. |
| Cellobiose | - | - |
| Trehalose | + | + |

Table 13

| Mycological Properties (Part 1) of Inventive Strain D66785 | |
|---|---|
| Morphology of Vegetative Cell | Egg-shape to Oblong-shape (3-8) × (6-10) μm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |
| Growth in Vitamin-Lacking Medium | Not growing |
| Growth in the presence of Cycloheximide (1000 ppm)<br>(100 ppm) | Not growing<br>Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 14

| Mycological Properties (Part 2) of Inventive Strain D66785 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | + | + |
| Galactose | + | + |
| Sucrose | + | + |
| Maltose | + | + |
| Lactose | - | - |
| Raffinose | + | + |
| Cellobiose | - | N.T. |
| Trehalose | + | - |

Table 15

| Mycological Properties (Part 1) of Inventive Strain D92764 | |
|---|---|
| Morphology of Vegetative Cell | Egg-shape to Oblong-shape (3-8) × (6-10) µm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |
| Growth in Vitamin-Lacking Medium | Not growing |
| Growth in the Presence of Cycloheximide (1000 ppm)<br>(100 ppm) | Not growing<br>Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 16

| Mycological Properties (Part 2) of Inventive Strain D92764 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | N.T. | + |
| Galactose | + | + |

Table 16 (continued)

| Mycological Properties (Part 2) of Inventive Strain D92764 | | |
|---|---|---|
| Item | Assimilation | Fermentation Ability |
| Sucrose | + | + |
| Maltose | + | + |
| Lactose | - | - |
| Raffinose | N.T. | - |
| Cellobiose | - | N.T. |
| Trehalose | + | N.T. |

Table 17

| Mycological Properties (Part 1) of Inventive Strain D80921 | |
|---|---|
| Morphology of Vegetative Cell | Egg-shape to Oblong-shape (3-8) × (6-10) µm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |
| Growth in Vitamin-Lacking Medium | Not growing |
| Growth in the presence of Cycloheximide (1000 ppm)<br>(100 ppm) | Not growing<br>Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 18

| Mycological Properties (Part 2) of Inventive Strain D80921 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | + | + |
| Galactose | + | + |
| Sucrose | + | + |
| Maltose | + | + |
| Lactose | - | - |
| Raffinose | + | + |
| Cellobiose | - | N.T. |
| Trehalose | + | - |

Table 19

| Mycological Properties (Part 1) of Inventive Strain D31735 | |
|---|---|
| Morphology of Vegetative Cell | Egg-shape to Oblong-shape (4-6) × (5-10) μm |
| Proliferation Style | Multipolar Budding |
| State of Growth | Excellently growing, formation of colonies (white, smooth, glossy) (25°C, 3 days, YPD medium) |
| Ascospore | Forming 1 to 4 spherical or oval ascospores, the ascospores not being divided. (25°C, 3 days, Adams medium) |
| Growth in Vitamin-Lacking Medium | Not growing |
| Growth in the presence of Cycloheximide (1000 ppm) (100 ppm) | Not growing<br>Not growing |
| Urea Degradation | Not degraded |
| Optimal Growth Conditions | pH: 5.0, temperature: 30°C, growing under aerobic conditions |
| Range of Growth | pH: 3.5-6.5, temperature: 5-40°C, growing under aerobic conditions |

Table 20

| Mycological Properties (Part 2) of Inventive Strain D31735 | | |
|---|---|---|
| Item | Assimilation | Fermentation Ability |
| (Nitrogen Source) | | |
| Nitrate | - | N.T. |
| Ethylamine | - | N.T. |
| Cadaverine | - | N.T. |
| (Carbon Source) | | |
| Glucose | + | + |
| Galactose | + | + |
| Sucrose | + | + |
| Maltose | + | + |
| Lactose | - | - |
| Raffinose | N.T. | + |
| Cellobiose | - | N.T. |
| Trehalose | + | N.T. |

Each of the strains has the mycological properties as described above, and the properties were checked with reference to "*The Yeasts, A Taxonomic Study (Fourth Edition)."* As a result, it was confirmed that any of the strains belong to *Saccharomyces cerevisiae*. Further, as mentioned above, each strain has a feature of exhibiting a high leavening ability in a dough having a high-sugar content to a super-high sugar content, and having drying tolerance; a feature of exhibiting a high leavening ability in a dough containing no sugar to a dough having a high sugar content, and having drying tolerance; a feature of exhibiting a high leavening ability in a dough containing no sugar to a dough having a low sugar content, and having drying tolerance; a feature of having a high freezing tolerance and/or floor resistance in a dough having a moderate-sugar content to a high-sugar content, and having drying tolerance; a feature of having a high freezing tolerance and/or floor resistance in a dough containing no sugar to a dough having a high-sugar content, and having drying tolerance; a feature of having a high freezing tolerance and/or floor resistance in a dough containing no sugar to a dough having a low-sugar content, and having drying tolerance; a feature of having a low-temperature sensitivity, and having drying tolerance. Therefore, any of the above-mentioned strains are not found in conventional strains, and are acknowledged as novel strains.

[Culture Conditions]

The process for culturing each of the above-mentioned yeasts is not particularly limited, as long as the process can be usually used in bread yeasts. In addition, an optimal growing pH, a pH range in which the yeast can grow, an optimal growth temperature, a temperature range in which the yeast can grow, and the like are the same as those of ordinary baker's yeasts. For instance, cells can be produced by molasses feeding cultivation method of theriac. The molasses can be substituted with other assimilable molasses. Also, the nitrogen source/phosphate source is not limited. Furthermore, a growth accelerating factor may be added thereto. The yeast of the present invention can be obtained as a compressed yeast by harvesting and washing yeast cells obtained after completion of culture, and subjecting the cells to dehydration.
The present invention also provides a dry yeast obtained by drying the above-mentioned yeast. The dry yeast as referred to herein is an yeast which is dried, preferably those having a water content in the cell of 5% by weight or less Here, the water content in the cell can be determined according to the above-mentioned method.
As mentioned above, it has been difficult to produce a dry yeast having a desired property for bread making. In the present invention, the process for drying an yeast is not particularly limited. For instance, in general, as a process for producing a dry yeast, there can be used a known process. For instance, a dry yeast can be obtained as follows. An aqueous emulsion of sorbitan fatty acid ester is added to a compressed yeast so as to have a content of 1.5% by weight in the dry yeast obtained therefrom, and mixed. The mixture is then passed through a screen having a mesh width of 0.5 mm with an extruder, to form into strands. The strands are subjected to a fluidized drying with a warm air using a fluidized dryer, setting the initial inlet temperature of the fluidized dryer at 44°C. The end point of drying is set at a point where the water content of the cell was 5% by weight or less, to give a dry yeast.
The dough of the present invention can be produced by kneading the above-mentioned yeast (yeast before drying) or dry yeast of the present invention together with various kinds of ingredients. The dough as referred to herein is a kneaded mixture produced by adding water to cereal flour as represented by wheat flour, and adding as desired thereto an additive including a fat or oil such as shortening; a saccharide such as sucrose, glucose, fructose or invert sugar; salt; an egg; a dairy product such as powdered skim milk, milk or fermented milk; an yeast food; an emulsifier such as monoglyceride; or the like. The dough refers to, but not particularly limited to, a bread dough. The dough of the present invention encompasses a dough for pie, a dough for steamed bread, a dough for pizza and the like. The above-mentioned cereal flour, water and the additive are not particularly limited, and known ones can be properly used. The yeast or dry yeast of the present invention has excellent leavening ability in various doughs of from those containing no sugar to those having a high-sugar concentration, and has drying tolerance. Also, as mentioned above, each of the yeasts has especially excellent properties in a dough having a specified sugar concentration range, from the viewpoint of the property for producing bread. Therefore, the yeast can meet the need for producing a general bread having any sugar concentrations. Also, the production of bread having even more excellent properties can be achieved by limiting the sugar concentration range. The content of the yeast or dry yeast of the present invention in a dough is not particularly limited. In the case of a raw yeast, its content is preferably from 1 to 6 parts by weight, based on 100 parts by weight of the flour. On the other hand, in the case of a dry yeast, its content is preferably from 0.5 to 3 parts by weight, based on 100 parts by weight of the flour.
The process for producing bread of the present invention is not particularly limited. For instance, the process includes scratch process, sponge and dough process, refrigerated dough process, and frozen dough process. It is effective in a scratch process that the yeast or dry yeast of the present invention is used in a composition wherein its sugar concentration is preferably from 0 to 40% by weight, more preferably from 0 to 30% by weight, from the viewpoint of exhibiting the desired effects of the present invention. Also, as to the yeast having freezing tolerance, it is effective to use the yeasts for a dough in refrigerated or frozen dough process. Here, the above-mentioned dough is one which becomes bread via a baking process in the process for producing these breads.
For instance, in a known frozen dough process, the frozen dough is usually produced by kneading ingredients at a relatively low temperature with the same dough composition as that of the so-called scratch dough other than the frozen dough to give a dough, subjecting the dough to pre-leavening (taking a floor time) for 30 to 120 minutes, dividing and molding the dough, and subjecting the dough to frozen storage. Next, the dough after frozen storage is thawed, subjected to final leavening and baked, thereby giving excellent bread having stable quality. Various materials have been known in the past on these doughs and production of breads, which can be properly referred. The kneading conditions, temperature conditions and the like are not particularly limited.
The present invention will be described by means of the following examples, without limiting the scope of the present invention thereto.

Example 1

The drying tolerance of each of yeasts *Saccharomyces cerevisiae* D75412, D20946, D46462, D66785, D92764, D80921 and D31735 of the present invention, was studied. Dry yeasts were produced from the yeasts of the present invention, and from a widely used yeast Kaneka Yeast Red (manufactured by KANEKA CORPORATION), low-temperature sensitive yeasts Kaneka Yeast White (manufactured by KANEKA CORPORATION) and Kaneka Yeast AL (manufactured by KANEKA CORPORATION), freezing-tolerant yeasts FD-I (manufactured by Company O), FD-II (manufactured by Company O) and YF (manufactured by Company J), which are commercially available, as the comparative controls, and the drying tolerances of the inventive dry yeasts were compared with those of the comparative controls. An aqueous emulsion of sorbitan fatty acid ester was added to each of the compressed yeasts in a proportion of 1.5% by weight of the dry yeast obtained therefrom, and mixed. The mixture was then passed through a screen having a mesh width of 0.5 mm with an extruder, to form into strands. The strands were dried in a fluidized bed dryer, wherein hot air was introduced into the dryer to keep the initial inlet temperature of 44°C. The drying was stopped when the water content of the yeast reached 5% by weight or lower, to give each of the dry yeasts.

The drying tolerance was evaluated by the method described in the (9) Drying Tolerance. The results are shown in Table 21. It can be seen that the remaining leavening ability ratios of the inventive dry yeasts are 0.70 or more, while the remaining leavening ability ratios of the commercially available yeasts are 0.50 or less, showing that the inventive dry yeasts have an excellent drying tolerance.

Table 21

| | Remaining Leavening Ability Ratio |
|---|---|
| Inventive Dry Yeast (D75412) | 0.80 |
| Inventive Dry Yeast (D20946) | 0.70 |
| Inventive Dry Yeast (D46462) | 0.81 |
| Inventive Dry Yeast (D66785) | 0.85 |
| Inventive Dry Yeast (D92764) | 0.85 |
| Inventive Dry Yeast (D80921) | 0.73 |
| Inventive Dry Yeast (D31735) | 0.70 |
| Dry Yeast Prepared from Commercially Available Compressed Yeast (Kaneka Yeast Red: Manufactured by KANEKA CORPORATION) | 0.50 |
| Dry Yeast Prepared from Commercially Available Low-Temperature Sensitive Compressed Yeast (Kaneka Yeast White: Manufactured by KANEKA CORPORATION) | 0.50 |
| Dry Yeast Prepared from Commercially Available Low-Temperature Sensitive Compressed Yeast (Kaneka Yeast AL: Manufactured by KANEKA CORPORATION) | 0.48 |
| Dry Yeast Prepared from Commercially Available Freezing-Tolerant Compressed Yeast (FD-I: Manufactured by Company O) | 0.04 |
| Dry Yeast Prepared from Commercially Available Freezing-Tolerant Compressed Yeast (FD-II: Manufactured by Company O) | 0.14 |
| Dry Yeast Prepared from Commercially Available Freezing-Tolerant Compressed Yeast (YF: Manufactured by Company J) | 0.14 |

Example 2

The remaining leavening ability ratios after drying were determined for the inventive yeast *Saccharomyces cerevisiae* D46462, at different drying temperatures. Dry yeasts were produced from the yeast of the present invention and a commercially available yeast Mauripan low sugar (manufactured by Company BP) used as a comparative control, and the drying tolerance of the inventive yeast was compared with that of the comparative control, at different drying temperatures. In the same manner as in Example 1, an aqueous emulsion of sorbitan fatty acid ester was added to each of the compressed yeasts so as to have a content of 1.5% by weight of the dry yeast obtained therefrom, and mixed. The mixture was then passed through a screen mesh having a pore size of 0.5 mm with an extruder, to give a thread-like product. The thread-like product was subjected to a fluidized drying with a warm air using a fluidized bed dryer, with setting the initial temperature of the inlet of the fluidized bed dryer at 44°C. Similarly, dry yeasts were obtained by varying inlet temperatures of the fluidized bed dryer from 50° to 65°C. The end point of drying was at a point where the water content of the cells was 5% by weight or less, to give each of the dry yeasts.

The drying tolerance was evaluated by the method described in the (9) Drying Tolerance. The results are shown in Table 22. It is shown that the remaining leavening ability ratios of the inventive dry yeast (D46462) and the dry yeast produced from Mauripan low sugar (manufactured by Company BP) are of the same level of 0.8 or so at a drying temperature of 44°C. However, the difference in remaining leavening ability ratio became greater as the temperature was increased, and at a drying temperature of 65°C, a remaining leavening ability ratio of the inventive dry yeast (D46462) was 0.64, while that of the dry yeast from Mauripan low sugar (manufactured by Company BP) was 0.51, thereby clearly showing that the inventive yeast D46462 has a stronger drying tolerance.

Table 22

| Comparison on Remaining Leavening Ability Ratio Due to Difference in Drying Temperature | | | | | |
|---|---|---|---|---|---|
| Drying Temperature | 44°C | 50°C | 55°C | 60°C | 65°C |
| Inventive Dry Yeast (D46462) | 0.81 | 0.72 | 0.71 | 0.69 | 0.64 |
| Commercially Available Dry Yeast Mauripan Low Sugar | 0.80 | 0.68 | 0.63 | 0.56 | 0.51 |

Example 3

When a dry yeast is produced from a compressed yeast, it is essential to add an emulsifier for the purpose of suppressing a decrease in the leavening ability of the yeast caused by drying. However, in recent years, consumers increasingly became natural oriented, and production of bread using as little additives as possible has been demanded. With different concentrations of an emulsifier added during drying, compressed yeasts were produced after cultivation from the yeast D46462 of the present invention and a commercially available yeast Mauripan low sugar (manufactured by Company BP) (a dry yeast manufactured article may be referred to herein as "commercially available dry yeast Mauripan low sugar") as the comparative control, and dry yeasts were produced therefrom. The drying tolerances were studied.

An aqueous emulsion of sorbitan fatty acid ester was added to each of the compressed yeasts so as to have a content of 0.8, 1.0, 1.2, 1.5 or 3.0% by weight of the dry yeast obtained therefrom, and mixed. The mixture was then passed through a screen mesh having a pore size of 0.5 mm with an extruder, to give a thread-like product. The thread-like product was subjected to a fluidized drying with a warm air using a fluidized bed dryer, with setting the initial temperature of the inlet of the fluidized bed dryer at 44°C. The end point of drying was set at a point where the water content of the cells was 5% by weight or less, to give each of the dry yeasts.

The drying tolerance was evaluated by the method described in the (9) Drying Tolerance. The results are shown in Table 23.

Table 23

| Comparison on Remaining Leavening Ability Ratio Due to Difference in Emulsifier Concentration | | | | | |
|---|---|---|---|---|---|
| Emulsifier Concentration (% by weight) | 0.8 | 1.0 | 1.2 | 1.5 | 3.0 |
| Inventive Dry Yeast (D46462) | 0.60 | 0.62 | 0.61 | 0.66 | 0.74 |
| Commercially Available Dry Yeast Mauripan Low Sugar | 0.35 | 0.46 | 0.57 | 0.61 | 0.62 |

As is clear from Table 23, the inventive dry yeast kept a remaining leavening ability ratio in a low sugar-content dough of 0.60 even at a concentration of added emulsifier of 0.8% by weight, while the dry yeast produced from the commercially available yeast had a drastically lowered leavening ability at a concentration of the added emulsifier of 1.0% by weight. It can be said from this finding that the content of an emulsifier added when the dry yeast is produced from the inventive yeast can be reduced, so that a dry yeast matching the earnest demands for natural-oriented foods can be obtained according to the present invention.

Example 4

The leavening ability in a dough having a sugar concentration of 30% by weight and the leavening ability in a dough having a sugar concentration of 40% by weight were determined for the yeast D75412 of the present invention. The leavening ability in a dough was determined by the method described in the above-mentioned (3) Leavening Ability in Dough Having Sugar Concentration of 30% by Weight and (4) Leavening Ability in Dough Having Sugar Concentration of 40% by Weight, respectively. The dry yeast was produced from the yeast D75412 of the present invention in the same manner as in Example 1. As the comparative controls, commercially available dry yeasts Saf-instant (Gold) (manufactured by Company S) and Fermipan Brown (manufactured by Company D), which have been considered to be suitable for producing sweet bread, were used. The results are shown in Table 24.

Table 24

| Leavening Ability in Dough Having Sugar Concentration of 30% by Weight and Leavening Ability in Dough Having Sugar Concentration of 40% by Weight | | |
|---|---|---|
| | Leavening Ability in Dough Having Sugar Concentration of 30% by Weight (ml) | Leavening Ability in Dough Having Sugar Concentration of 40% by Weight (ml) |
| Inventive Dry Yeast (D75412) | 261 | 111 |
| Commercially Available Dry Yeast Saf-instant (Gold) | 192 | 66 |
| Commercially Available Dry Yeast Fermipan Brown | 166 | 59 |

As shown in Table 24, it is seen that in a dough having a sugar concentration of 30% by weight, the leavening ability of the inventive dry yeast D75412 is 261 ml, while the leavening abilities of the commercially available dry yeasts are less than 200 ml, showing that the inventive dry yeast has a more excellent leavening ability. Further, it is seen that in a dough having a sugar concentration of 40% by weight, the leavening ability of the inventive dry yeast (D75412) is 111 ml, while the leavening abilities of the commercially available dry yeasts are less than 70 ml, showing that the inventive dry yeast also has a more excellent leavening ability in a dough having a sugar concentration of 40% by weight. The results for the leavening abilities show that the inventive dry yeast is most suitable for doughs for sweet bread containing sugar in a large amount, such as a bean-jam bun.

Example 5

The leavening ability in a dough having a sugar concentration of 0% by weight and the leavening ability in a dough having a sugar concentration of 30% by weight were determined for the yeast D20946 of the present invention. The leavening ability in a dough was determined by the method described in the above-mentioned (1) Leavening Ability in Dough Having Sugar Concentration of 0% by Weight and (3) Leavening Ability in Dough Having Sugar Concentration of 30% by Weight, respectively. The dry yeast was produced from the yeast D20946 of the present invention in the same manner as in Example 1. The leavening ability in a dough having a sugar concentration of 0% by weight and the leavening ability in a dough having a sugar concentration of 30% by weight were determined for two commercially available dry yeasts Saf-instant (RED) (manufactured by Company S) and Fermipan RED (manufactured by Company D), which have been considered to be suitable for producing white bread, and two commercially available dry yeasts Saf-instant (Gold) (manufactured by Company S) and Fermipan Brown (manufactured by Company D), which have been considered to be suitable for producing sweet bread, a total of four commercially available yeasts which are used as the comparative controls. The results are shown in Table 25.

Table 25

| Leavening Ability in Dough Having Sugar Concentration of 0% by Weight and Leavening Ability in Dough Having Sugar Concentration of 30% by Weight | | |
|---|---|---|
| | Leavening Ability in Dough Having Sugar Concentration of 0% by Weight (ml) | Leavening Ability in Dough Having Sugar Concentration of 30% by Weight (ml) |
| Inventive Dry Yeast (D20946) | 196 | 236 |
| Commercially Available Dry Yeast Saf-instant (RED) | 175 | 27 |
| Commercially Available Dry Yeast Fermipan RED | 198 | 90 |
| Commercially Available Dry Yeast Saf-instant (Gold) | 98 | 200 |
| Commercially Available Dry Yeast Fermipan Brown | 134 | 188 |

As shown in Table 25, the leavening abilities of the commercially available dry yeasts were less than 100 ml in a dough having a sugar concentration of 30% by weight even if the dry yeasts had a sufficiently high leavening ability of 170 ml or more in a dough having a sugar concentration of 0% by weight, showing that the commercially available dry yeasts are not suitable for producing sweet bread having high sugar concentrations. Also, the commercially available dry yeasts having a leavening ability of 180 ml or more in a dough having a sugar concentration of 30% by weight and being suitable for producing sweet bread had a leavening ability of less than 140 ml in a dough having a sugar concentration of 0% by weight, showing that they are not suitable for producing French bread.

On the other hand, the inventive dry yeast (D20946) had a sufficiently high leavening ability of 196 ml or more in a dough having a sugar concentration of 0% by weight, and a sufficiently high leavening ability of 236 ml or more in a dough having a sugar concentration of 30% by weight, showing that the inventive dry yeast has a leavening ability sufficient for production of from a non-sugar dough for French bread or the like to a dough having a high sugar content for sweet bread or the like.

Example 6

The leavening ability in a dough having a sugar concentration of 0% by weight and the leavening ability in a dough having a sugar concentration of 5% by weight were determined for the yeast D46462 of the present invention. The leavening ability in a dough was determined by the method described in the above-mentioned (1) Leavening Ability in Dough Having Sugar Concentration of 0% by Weight and (2) Leavening Ability in Dough Having Sugar Concentration of 5% by Weight, respectively. The dry yeast was produced from the yeast D46462 of the present invention in the same manner as in Example 1. The leavening ability in a dough having a sugar concentration of 0% by weight and the leavening ability in a dough having a sugar concentration of 5% by weight were determined for commercially available dry yeasts Bruggeman Blue (manufactured by Company BR), Saf-instant (RED) (manufactured by Company S) and Mauripan low sugar (manufactured by BP), which have been considered to be suitable for producing white bread, as the comparative controls. The results are shown in Table 26.

Table 26

| Leavening Ability in Dough Having Sugar Concentration of 0% by Weight and Leavening Ability in Dough Having Sugar Concentration of 5% by Weight | | |
|---|---|---|
| | Leavening Ability in Dough Having Sugar Concentration of 0% by Weight (ml) | Leavening Ability in Dough Having Sugar Concentration of 5% by Weight (ml) |
| Inventive Dry Yeast (D46462) | 244 | 193 |
| Commercially Available Dry Yeast Bruggeman Blue | 208 | 134 |
| Commercially Available Dry Yeast Saf-instant (RED) | 211 | 142 |
| Commercially Available Dry Yeast Mauripan low sugar | 179 | 153 |

As shown in Table 26, the commercially available dry yeasts had a leavening ability of less than 220 ml in a dough having a sugar concentration of 0% by weight, and a leavening ability of less than 160 ml in a dough having a sugar concentration of 5% by weight. On the other hand, the inventive dry yeast D46462 had a leavening ability of 244 ml in a dough having a sugar concentration of 0% by weight, and a leavening ability of 193 ml in a dough having a sugar concentration of 5% by weight, showing that the inventive dry yeast have a far higher leavening ability as compared to those of the commercially available dry yeasts.

Example 7

The amounts of gas generated in a dough after second kneading (*hongone*) and after subjecting to the final proof (*hoiro*) (the amount of gas generated during the final proof) in the sugar-added sponge and dough process were determined for the inventive yeasts D75412 and D20946. As the comparative controls, commercially available dry yeasts Saf-instant (Gold) (manufactured by Company S) and Fermipan Brown (manufactured by Company D), which have been considered to be suitable for producing sweet bread, were used. The amount of gas generated in a dough after second kneading was determined by the method described in the above-mentioned (5) Amount of Gas Generated in Dough After Second Kneading (*Hongone*). Similarly, a dough after molding was divided into 50 g-portions, and thereafter the amount (ml) of gas generated was determined using Fermograph (manufactured by ATTO Co. Ltd.

Japan) at 30°C for 2 hours. This amount of gas generated was defined in a dough after subjecting to the final proof. Further, the ratio of the volume of bread to the weight of the bread after baking was determined as its specific volume (ml/g). The results obtained are shown in Table 27.

Table 27

| Amount of Gas Generated in Dough After Second Kneading and After Subjecting to Final Proof in Sugar-Added Sponge and Dough Process, and Specific Volume of Bread After Baking | | | |
|---|---|---|---|
| | Amount of Gas Generated After Second Kneading (ml) | Amount of Gas Generated After Subjecting to Final Proof (ml) | Specific Volume of Bread (ml/g) |
| Inventive Dry Yeast (D75412) | 193 | 216 | 5.89 |
| Inventive Dry Yeast (D20946) | 176 | 192 | 5.54 |
| Commercially Available Dry Yeast Saf-instant (Gold) | 116 | 130 | 4.59 |
| Commercially Available Dry Yeast Fermipan Brown | 90 | 101 | 4.17 |

As shown in Table 27, the amount of gas generated after second kneading of the inventive dry yeast (D75412) was as remarkably high as 193 ml and that of the inventive dry yeast (D20946) was as remarkably high as 176 ml, while the amount of gas generated of any of the commercially available dry yeasts was 120 ml or less. The high level of the amounts of the gas generated after second kneading caused a difference in the amount of gas generated after subsequently subjecting to the final proof. The finally baked bread using a commercially available dry yeast was poorly leavened, and its specific volume was as low as 4.6 ml/g or less. On the other hand, the baked bread using the inventive dry yeast was remarkably large in size. The specific volume of the inventive dry yeast (D75412) was 5.89 ml/g, and that of the inventive dry yeast (D20946) was 5.54 ml/g.

Example 8

The freezing tolerance was studied for the inventive yeasts D92764 and D80921. The freezing tolerance was evaluated by the method described in the above-mentioned (6) Freezing Tolerance. A dry yeast was produced from each of the yeasts of the present invention in the same manner as in Example 1. As the comparative controls, commercially available dry yeasts Saf-instant (Red) (manufactured by Company S) and Fermipan Red (manufactured by Company D) were used. In the evaluation of the freezing tolerance, the concentration of sugar in a dough was 0% by weight. The results are shown in Table 28.

Table 28

| Leavening Ability in Dough Having Sugar Concentration of 0% by Weight Before and After 4-Week Frozen Storage Following 60-minute Floor Time | | | |
|---|---|---|---|
| Dough Having Sugar Concentration of 0% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D92764) | 120 | 110 | 0.92 |
| Inventive Dry Yeast (D80921) | 113 | 110 | 0.97 |
| Commercially Available Dry Yeast Saf-instant (Red) | 106 | 91 | 0.86 |

Table 28 (continued)

| Leavening Ability in Dough Having Sugar Concentration of 0% by Weight Before and After 4-Week Frozen Storage Following 60-minute Floor Time | | | |
|---|---|---|---|
| Dough Having Sugar Concentration of 0% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Commercially Available Dry Yeast Fermipan Red | 102 | 65 | 0.64 |

As shown in Table 28, it can be seen that the leavening ability after frozen storage of the inventive dry yeast (D92764) is 110 ml and that of the inventive dry yeast (D80921) is 110 ml, while those of the commercially available dry yeasts are less than 100 ml, showing that the inventive dry yeasts have an excellent leavening ability after frozen storage. Further, it can be seen that the leavening ability ratio after frozen storage to leavening ability before frozen storage of the inventive dry yeast (D92764) is 0.92 and that of the inventive dry yeast (D80921) is 0.97, while those of the commercially available dry yeasts are less than 0.88, indicating that the inventive dry yeasts have an excellent freezing tolerance.

In addition, the leavening abilities after frozen storage were determined when a frozen storage time period was set to 1 week, 2 weeks and 4 weeks. The results are shown in Figure 1. As is clear from Figure 1, under the conditions in which a dough having a sugar concentration of 0% by weight is subjected to a floor time of 60 minutes, the inventive dry yeasts showed a marked freezing tolerance in any of the frozen storage time periods of 1 week, 2 weeks and 4 weeks, as compared to those of the commercially available dry yeasts.

Example 9

The freezing tolerance was studied for the inventive yeast D80921. The freezing tolerance was evaluated by the method described in the above-mentioned (6) Freezing Tolerance. A dry yeast was produced from the yeast of the present invention in the same manner as in Example 1. As the comparative controls, commercially available dry yeasts Saf-instant (Red) (manufactured by Company S) and Fermipan Red (manufactured by Company D) were used. In the evaluation of the freezing tolerance, the concentration of sugar in a dough was 3% by weight. The results are shown in Table 29.

Table 29

| Leavening Ability in Dough Having Sugar Concentration of 3% by Weight Before and After 4-Week Frozen Storage Following 60-minute Floor Time | | | |
|---|---|---|---|
| Dough Having Sugar Concentration of 3% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D80921) | 135 | 79 | 0.58 |
| Commercially Available Dry Yeast Saf-instant (Red) | 152 | 47 | 0.31 |
| Commercially Available Dry Yeast Fermipan Red | 145 | 26 | 0.18 |

As shown in Table 29, it can be seen that the leavening ability after frozen storage of the inventive dry yeast (D80921) is 79 ml, while those of the commercially available dry yeasts are less than 50 ml, showing that the inventive dry yeast has an excellent leavening ability after frozen storage. Further, it can be seen that the leavening ability ratio after frozen storage to leavening ability before frozen storage of the inventive dry yeast is 0.58, while those of the commercially available dry yeasts are less than 0.35, showing that the inventive dry yeast has an excellent freezing tolerance.

In addition, the leavening abilities after frozen storage were determined by setting a frozen storage time period to 1 week, 2 weeks and 4 weeks. The results are shown in Figure 2. As is clear from Figure 2, under the conditions in which a dough having a sugar concentration of 3% by weight is subjected to a floor time of 60 minutes, the inventive dry yeast showed a marked freezing tolerance in any of the frozen storage time periods of 1 week, 2 weeks and 4 weeks, as compared to those of the commercially available dry yeasts.

Example 10

The freezing tolerance was studied for the inventive yeast D92764. The freezing tolerance was evaluated by the method described in the above-mentioned (6) Freezing Tolerance. A dry yeast was produced from each of the yeasts of the present invention in the same manner as in Example 1. As the comparative controls, commercially available dry yeasts Saf-instant (Red) (manufactured by Company S) and Fermipan Red (manufactured by Company D) were used. In the evaluation of the freezing tolerance, the concentration of sugar in a dough was 5% by weight. The results are shown in Table 30.

Table 30

| Leavening Ability in Dough Having Sugar Concentration of 5% by Weight Before and After 4-Week Frozen Storage Following 60-minute Floor Time | | | |
|---|---|---|---|
| Dough Having Sugar Concentration of 5% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D92764) | 127 | 91 | 0.72 |
| Commercially Available Dry Yeast Saf-instant (Red) | 174 | 63 | 0.36 |
| Commercially Available Dry Yeast Fermipan Red | 177 | 65 | 0.36 |

As shown in Table 30, it can be seen that the leavening ability after frozen storage of the inventive dry yeast is 91 ml, while those of the commercially available dry yeasts are less than 70 ml, showing that the inventive dry yeast has an excellent leavening ability after frozen storage. Further, it can be seen that the leavening ability ratio after frozen storage to leavening ability before frozen storage of the inventive dry yeast is 0.72, while those of the commercially available dry yeasts are less than 0.40, showing that the inventive dry yeast has an excellent freezing tolerance.

In addition, the leavening abilities after frozen storage were determined by setting a frozen storage time period to 1 week, 2 weeks and 4 weeks. The results are shown in Figure 3. As is clear from Figure 3, under the conditions in which a dough having a sugar concentration of 5% by weight is subjected to a floor time of 60 minutes, the inventive dry yeast showed a marked freezing tolerance in any of the frozen storage time periods of 1 week, 2 weeks and 4 weeks, as compared to those of the commercially available dry yeasts.

Example 11

The freezing tolerance was studied for the inventive yeasts D92764 and D66785. The freezing tolerance was evaluated by the method described in the above-mentioned (6) Freezing Tolerance. A dry yeast was produced from each of the yeasts of the present invention in the same manner as in Example 1. As the comparative controls, commercially available dry yeasts Saf-instant (Red) and Saf-instant (Gold) (manufactured by Company S), and Fermipan Red and Fermipan Brown (manufactured by Company D) were used. In the evaluation of the freezing tolerance, the concentration of sugar in a dough was 10% by weight. The results are shown in Table 31.

Table 31

| Leavening Ability in Dough Having Sugar Concentration of 10% by Weight Before and After 4-Week Frozen Storage Following 60-minute Floor Time | | | |
|---|---|---|---|
| Dough Having Sugar Concentration of 10% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D92764) | 163 | 112 | 0.69 |
| Inventive Dry Yeast (D66785) | 194 | 109 | 0.56 |

Table 31 (continued)

| Leavening Ability in Dough Having Sugar Concentration of 10% by Weight Before and After 4-Week Frozen Storage Following 60-minute Floor Time | | | |
|---|---|---|---|
| Dough Having Sugar Concentration of 10% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Commercially Available Dry Yeast Saf-instant (Red) | 185 | 80 | 0.43 |
| Commercially Available Dry Yeast Fermipan Red | 182 | 49 | 0.27 |
| Commercially Available Dry Yeast Saf-instant (Gold) | 166 | 42 | 0.25 |
| Commercially Available Dry Yeast Fermipan Brown | 178 | 29 | 0.16 |

As shown in Table 31, it can be seen that the leavening ability after frozen storage of the inventive dry yeast (D92764) is 112 ml and that of the inventive dry yeast (D66785) is 109 ml, while those of the commercially available dry yeasts are 80 ml or less, showing that the inventive dry yeasts have an excellent leavening ability after frozen storage. Further, it can be seen that the leavening ability ratio after frozen storage to leavening ability before frozen storage of the inventive dry yeast (D92764) is 0.69 and that of the inventive dry yeast (D66785) is 0.56, while those of the commercially available dry yeasts are less than 0.45, showing that the inventive dry yeasts have an excellent freezing tolerance.

In addition, the leavening abilities after frozen storage were determined by setting a frozen storage time period to 1 week, 2 weeks and 4 weeks. The results are shown in Figure 4. As is clear from Figure 4, under the conditions in which a dough having a sugar concentration of 10% by weight is subjected to a floor time of 60 minutes, the inventive dry yeasts showed a marked freezing tolerance in any of the frozen storage time periods of 1 week, 2 weeks and 4 weeks, as compared to those of the commercially available dry yeasts.

Example 12

The freezing tolerance was studied for the inventive yeast D66785. The freezing tolerance was evaluated by the method described in the above-mentioned (6) Freezing Tolerance. A dry yeast was produced from the yeast of the present invention in the same manner as in Example 1. As the comparative controls, commercially available dry yeasts Saf-instant (Gold) (manufactured by Company S) and Fermipan Brown (manufactured by Company D) were used. In the evaluation of the freezing tolerance, the concentration of sugar in a dough was 15% by weight. The results are shown in Table 32.

Table 32

| Leavening Ability in Dough Having Sugar Concentration of 15% by weight Before and After 4-Week Frozen Storage Following 60-minute Floor Time | | | |
|---|---|---|---|
| Dough Having Sugar Concentration of 15% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D66785) | 200 | 132 | 0.66 |
| Commercially Available Dry Yeast Saf-instant (Gold) | 187 | 62 | 0.33 |
| Commercially Available Dry Yeast Fermipan Brown | 192 | 37 | 0.19 |

As shown in Table 32, it can be seen that the leavening ability after frozen storage of the inventive dry yeast (D66785) is 132 ml, while those of the commercially available dry yeasts are 62 ml or less, showing that the inventive dry yeast has an excellent leavening ability after frozen storage. Further, it can be seen that the leavening ability ratio after frozen storage to leavening ability before frozen storage of the inventive dry yeast (D66785) is 0.66, while those of the commercially available dry yeasts are less than 0.35, showing that the inventive dry yeast has an excellent freezing tolerance.

In addition, the leavening abilities after frozen storage were determined by setting a frozen storage time period to 1 week, 2 weeks and 4 weeks. The results are shown in Figure 5. As is clear from Figure 5, under the conditions in which a dough having a sugar concentration of 15% by weight is subjected to a floor time of 60 minutes, the inventive dry yeast showed a marked freezing tolerance in any of the frozen storage time periods of 1 week, 2 weeks and 4 weeks, as compared to those of the commercially available dry yeasts.

Example 13

The freezing tolerance was studied for the inventive yeasts D92764 and D66785. The freezing tolerance was evaluated by the method described in the above-mentioned (6) Freezing Tolerance. A dry yeast was produced from each of the yeasts of the present invention in the same manner as in Example 1. As the comparative controls, commercially available dry yeasts Saf-instant (Gold) (manufactured by Company S), and Fermipan Brown (manufactured by Company D) were used. In the evaluation of the freezing tolerance, the concentration of sugar in a dough was 25% by weight. The results are shown in Tables 33 and 34.

Table 33

| Leavening Ability in Dough Having Sugar Concentration of 25% by wt. Before and After 4-Week Frozen Storage Following 60-minute Floor Time | | | |
|---|---|---|---|
| Dough Having Sugar Concentration of 25% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D92764) | 191 | 138 | 0.72 |
| Inventive Dry Yeast (D66785) | 186 | 145 | 0.78 |
| Commercially Available Dry Yeast Saf-instant (Gold) | 174 | 120 | 0.69 |
| Commercially Available Dry Yeast Fermipan Brown | 162 | 109 | 0.67 |

Table 34

| Leavening Ability in Dough Having Sugar Concentration of 25% by wt. Before and After 4-Week Frozen Storage Following 90-minute Floor Time | | | |
|---|---|---|---|
| Dough Having Sugar Concentration of 25% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Inventive Dry Yeast (D92764) | 182 | 65 | 0.36 |
| Inventive Dry Yeast (D66785) | 186 | 119 | 0.64 |
| Commercially Available Dry Yeast Saf-instant (Gold) | 168 | 29 | 0.18 |

Table 34 (continued)

| Leavening Ability in Dough Having Sugar Concentration of 25% by wt. Before and After 4-Week Frozen Storage Following 90-minute Floor Time | | | |
|---|---|---|---|
| Dough Having Sugar Concentration of 25% by Weight | Leavening Ability Before Frozen Storage (ml/120 min/ 20 g Dough) | Leavening Ability After Frozen Storage (ml/120 min/ 20 g Dough) | Leavening Ability Ratio (After Frozen Storage/ Before Frozen Storage) |
| Commercially Available Dry Yeast Fermipan Brown | 161 | 38 | 0.23 |

As shown in Tables 33 and 34, it can be seen that when the floor time is 60 minutes, the leavening ability after frozen storage of the inventive dry yeast (D92764) is 138 ml and that of the inventive dry yeast (D66785) is 145 ml, while those of the commercially available dry yeasts are 120 ml or less, and that when the floor time is 90 minutes, the leavening ability after frozen storage of the inventive dry yeast (D92764) is 65 ml and that of the inventive dry yeast (D66785) is 119 ml, while those of the commercially available dry yeasts are 40 ml or less, showing that the inventive dry yeasts have an excellent leavening ability after frozen storage. Further, it can be seen that when the floor time is 60 minutes, the leavening ability ratio after frozen storage to leavening ability before frozen storage of the inventive dry yeast (D92764) is 0.72 and that of the inventive dry yeast (D66785) is 0.78, while those of the commercially available dry yeasts are less than 0.70, and that when the floor time is 90 minutes, the leavening ability ratio after frozen storage to leavening ability before frozen storage of the inventive dry yeast (D92764) is 0.36 and that of the inventive dry yeast (D66785) is 0.64, while those of the commercially available dry yeasts are less than 0.25, showing that the inventive dry yeasts have an excellent freezing tolerance.

In addition, the leavening abilities after frozen storage were determined by setting a frozen storage time period to 1 week, 2 weeks and 4 weeks. The results are shown in Figure 6. As is clear from Figure 6, under the conditions in which a dough having a sugar concentration of 25% by weight is subjected to a floor time of 90 minutes, the inventive dry yeasts showed a marked freezing tolerance in any of the frozen storage time periods of 1 week, 2 weeks and 4 weeks, as compared to those of the commercially available dry yeasts.

Example 14

The floor resistance was studied for the inventive yeasts D92764 and D80921. The floor resistance was evaluated by the method described in the above-mentioned (7) Floor Resistance. A dry yeast was produced from each of the yeasts of the present invention in the same manner as in Example 1. As the comparative controls, commercially available dry yeasts Saf-instant (Red) (manufactured by Company S), and Fermipan Red (manufactured by Company D) were used. In the evaluation of the floor resistance, the concentration of sugar in a dough was 0% by weight. The results are shown in Table 35.

Table 35

| Floor Resistance After 4-Week Frozen Storage of Dough Having Sugar Concentration of 0% by Weight | | | |
|---|---|---|---|
| Dough Having Sugar Concentration of 0% by Weight | Leavening Ability in Case of 0-Minute Floor Time (ml/120 min/ 20 g Dough) | Leavening Ability in Case of 60-Minute Floor Time (ml/120 min/ 20 g Dough) | Leavening Ability Ratio (60-Minute Floor Time/ 0-Minute Floor Time) |
| Inventive Dry Yeast (D92764) | 110 | 110 | 1.00 |
| Inventive Dry Yeast (D80921) | 122 | 110 | 0.90 |
| Commercially Available Dry Yeast Saf-instant (Red) | 131 | 91 | 0.70 |
| Commercially Available Dry Yeast Fermipan Red | 142 | 65 | 0.46 |

As shown in Table 35, it can be seen that the leavening ability ratio expressing the floor resistance (leavening ability in the case of 60-minute floor time/leavening ability in the case of 0-minute floor time) of the inventive dry yeast (D92764) is 1.00 and that of the inventive dry yeast (D80921) is 0.90, while those of the commercially available dry yeasts are 0.70 or less, showing that the inventive dry yeasts have an excellent floor resistance.

In addition, the floor resistance at each point was determined by setting a frozen storage time period to 1 week, 2 weeks and 4 weeks. The results are shown in Figure 7. As is clear from Figure 7, in a dough having a sugar concentration of 0% by weight, the inventive dry yeasts showed a marked floor resistance in any of the frozen storage time periods of 1 week, 2 weeks and 4 weeks, as compared to those of the commercially available dry yeasts.

Example 15

The floor resistance was studied for the inventive yeast D80921. The floor resistance was evaluated by the method described in the above-mentioned (7) Floor Resistance. A dry yeast was produced from the yeast of the present invention in the same manner as in Example 1. As the comparative controls, commercially available dry yeasts Saf-instant (Red) (manufactured by Company S), and Fermipan Red (manufactured by Company D) were used. In the evaluation of the floor resistance, the concentration of sugar in a dough was 3% by weight. The results are shown in Table 36.

Table 36

| Floor Resistance After 4-Week Frozen Storage of Dough Having Sugar Concentration of 3% by Weight | | | |
|---|---|---|---|
| Dough Having Sugar Concentration of 3% by Weight | Leavening Ability in Case of 0-Minute Floor Time (ml/120 min/ 20 g Dough) | Leavening Ability in Case of 60-Minute Floor Time (ml/120 min/ 20 g Dough) | Leavening Ability Ratio (60-Minute Floor Time/ 0-Minute Floor Time) |
| Inventive Dry Yeast (D80921) | 145 | 79 | 0.54 |
| Commercially Available Dry Yeast Saf-instant (Red) | 163 | 47 | 0.29 |
| Commercially Available Dry Yeast Fermipan Red | 176 | 26 | 0.15 |

As shown in Table 36, it can be seen that the leavening ability ratio expressing the floor resistance (leavening ability in the case of 60-minute floor time/leavening ability in the case of 0-minute floor time) of the inventive dry yeast (D80921) is 0.54, while those of the commercially available dry yeasts are less than 0.30, showing that the inventive dry yeasts have an excellent floor resistance.

In addition, the floor resistance at each point was determined by setting a frozen storage time period to 1 week, 2 weeks and 4 weeks. The results are shown in Figure 8. As is clear from Figure 8, in a dough having a sugar concentration of 3% by weight, the inventive dry yeast showed a marked floor resistance in any of the frozen storage time periods of 1 week, 2 weeks and 4 weeks, as compared to those of the commercially available dry yeasts.

Example 16

The floor resistance was studied for the inventive yeast D92764. The floor resistance was evaluated by the method described in the above-mentioned (7) Floor Resistance. A dry yeast was produced from the yeast of the present invention in the same manner as in Example 1. As the comparative controls, commercially available dry yeasts Saf-instant (Red) (manufactured by Company S), and Fermipan Red (manufactured by Company D) were used. In the evaluation of the floor resistance, the concentration of sugar in a dough was 5% by weight. The results are shown in Table 37.

Table 37

| Floor Resistance After 4-Week Frozen Storage of Dough Having Sugar Concentration of 5% by Weight | | | |
|---|---|---|---|
| Dough Having Sugar Concentration of 5% by Weight | Leavening Ability in Case of 0-Minute Floor Time (ml/120 min/ 20 g Dough) | Leavening Ability in Case of 60-Minute Floor Time (ml/120 min/ 20 g Dough) | Leavening Ability Ratio (60-Minute Floor Time/ 0-Minute Floor Time) |
| Inventive Dry Yeast (D92764) | 136 | 91 | 0.67 |

Table 37 (continued)

| Floor Resistance After 4-Week Frozen Storage of Dough Having Sugar Concentration of 5% by Weight | | | |
|---|---|---|---|
| Dough Having Sugar Concentration of 5% by Weight | Leavening Ability in Case of 0-Minute Floor Time (ml/120 min/ 20 g Dough) | Leavening Ability in Case of 60-Minute Floor Time (ml/120 min/ 20 g Dough) | Leavening Ability Ratio (60-Minute Floor Time/ 0-Minute Floor Time) |
| Commercially Available Dry Yeast Saf-instant (Red) | 131 | 63 | 0.48 |
| Commercially Available Dry Yeast Fermipan Red | 146 | 65 | 0.45 |

As shown in Table 37, it can be seen that the leavening ability ratio expressing the floor resistance (leavening ability in the case of 60-minute floor time/leavening ability in the case of 0-minute floor time) of the inventive dry yeast (D92764) is 0.67, while those of the commercially available dry yeasts are less than 0.50, showing that the inventive dry yeast has an excellent floor resistance.

In addition, the floor resistance at each point was determined by setting a frozen storage time period to 1 week, 2 weeks and 4 weeks. The results are shown in Figure 9. As is clear from Figure 9, in a dough having a sugar concentration of 5% by weight, especially when the frozen storage period is over a long time period (2 weeks or 4 weeks), the inventive dry yeast showed a marked floor resistance, as compared to those of the commercially available dry yeasts.

Example 17

The floor resistance was studied for the inventive yeasts D92764 and D66785. The floor resistance was evaluated by the method described in the above-mentioned (7) Floor Resistance. A dry yeast was produced from each of the yeasts of the present invention in the same manner as in Example 1. As the comparative controls, commercially available dry yeasts Saf-instant (Red) and Saf-instant (Gold) (manufactured by Company S), and Fermipan Red and Fermipan Brown (manufactured by Company D) were used. In the evaluation of the floor resistance, the concentration of sugar in a dough was 10% by weight. The results are shown in Table 38.

Table 38

| Floor Resistance After 4-Week Frozen Storage of Dough Having Sugar Concentration of 10% by Weight | | | |
|---|---|---|---|
| Dough Having Sugar Concentration of 10% by Weight | Leavening Ability in Case of 30-Minute Floor Time (ml/120 min/ 20 g Dough) | Leavening Ability in Case of 90-Minute Floor Time (ml/120 min/ 20 g Dough) | Leavening Ability Ratio (90-Minute Floor Time/ 30-Minute Floor Time) |
| Inventive Dry Yeast (D92764) | 152 | 69 | 0.46 |
| Inventive Dry Yeast (D66785) | 152 | 59 | 0.39 |
| Commercially Available Dry Yeast Saf-instant (Red) | 148 | 19 | 0.13 |
| Commercially Available Dry Yeast Fermipan Red | 124 | 14 | 0.11 |
| Commercially Available Dry Yeast Saf-instant (Gold) | 140 | 18 | 0.13 |
| Commercially Available Dry Yeast Fermipan Brown | 95 | 13 | 0.14 |

As shown in Table 38, it can be seen that the leavening ability ratio expressing the floor resistance (leavening ability in the case of 90-minute floor time/leavening ability in the case of 30-minute floor time) of the inventive dry yeast (D92764) is 0.45 and that of the inventive dry yeast (D66785) is 0.39, while those of the commercially available dry yeasts are less than 0.15, showing that the inventive dry yeasts have an excellent floor resistance.

In addition, the floor resistance at each point was determined by setting a frozen storage time periods to 1 week, 2 weeks and 4 weeks. The results are shown in Figure 10. As is clear from Figure 10, in a dough having a sugar concentration of 10% by weight, the inventive dry yeasts showed a marked floor resistance in any of the frozen storage time periods of 1 week, 2 weeks and 4 weeks, as compared to those of the commercially available dry yeasts.

Example 18

The floor resistance was studied for the inventive yeast D66785. The floor resistance was evaluated by the method described in the above-mentioned (7) Floor Resistance. A dry yeast was produced from the yeast of the present invention in the same manner as in Example 1. As the comparative controls, commercially available dry yeasts Saf-instant (Gold) (manufactured by Company S), and Fermipan Brown (manufactured by Company D) were used. In the evaluation of the floor resistance, the concentration of sugar in a dough was 15% by weight. The results are shown in Table 39.

Table 39

| Floor Resistance After 4-Week Frozen Storage of Dough Having Sugar Concentration of 15% by Weight | | | |
|---|---|---|---|
| Dough Having Sugar Concentration of 15% by Weight | Leavening Ability in Case of 30-Minute Floor Time (ml/120 min/ 20 g Dough) | Leavening Ability in Case of 90-Minute Floor Time (ml/120 min/ 20 g Dough) | Leavening Ability Ratio (90-Minute Floor Time/ 30-Minute Floor Time) |
| Inventive Dry Yeast (D66785) | 172 | 83 | 0.48 |
| Commercially Available Dry Yeast Saf-instant (Gold) | 151 | 23 | 0.15 |
| Commercially Available Dry Yeast Fermipan Brown | 148 | 20 | 0.14 |

As shown in Table 39, it can be seen that the leavening ability ratio expressing the floor resistance (leavening ability in the case of 90-minute floor time/leavening ability in the case of 30-minute floor time) of the inventive dry yeast (D66785) is 0.48, while those of the commercially available dry yeasts are 0.15 or less, showing that the inventive dry yeast has an excellent floor resistance.

In addition, the floor resistance at each point was determined by setting a frozen storage time period to 1 week, 2 weeks and 4 weeks. The results are shown in Figure 11. As is clear from Figure 11, in a dough having a sugar concentration of 15% by weight, the inventive dry yeast showed a marked floor resistance in any of the frozen storage time periods of 1 week, 2 weeks and 4 weeks, as compared to those of the commercially available dry yeasts.

Example 19

The floor resistance was studied for the inventive yeasts D92764 and D66785. The floor resistance was evaluated by the method described in the above-mentioned (7) Floor Resistance. A dry yeast was produced from each of the yeasts of the present invention in the same manner as in Example 1. As the comparative controls, commercially available dry yeasts Saf-instant (Gold) (manufactured by Company S), and Fermipan Brown (manufactured by Company D) were used. In the evaluation of the floor resistance, the concentration of sugar in a dough was 25% by weight. The results are shown in Table 40.

Table 40

| Floor Resistance After 4-Week Frozen Storage of Dough Having Sugar Concentration of 25% by Weight | | | |
|---|---|---|---|
| Dough Having Sugar Concentration of 25% by Weight | Leavening Ability in Case of 30-Minute Floor Time (ml/120 min/ 20 g Dough) | Leavening Ability in Case of 90-Minute Floor Time (ml/120 min/ 20 g Dough) | Leavening Ability Ratio (90-Minute Floor Time/ 30-Minute Floor Time) |
| Inventive Dry Yeast (D92764) | 171 | 65 | 0.38 |

Table 40 (continued)

| Floor Resistance After 4-Week Frozen Storage of Dough Having Sugar Concentration of 25% by Weight | | | |
|---|---|---|---|
| Dough Having Sugar Concentration of 25% by Weight | Leavening Ability in Case of 30-Minute Floor Time (ml/120 min/ 20 g Dough) | Leavening Ability in Case of 90-Minute Floor Time (ml/120 min/ 20 g Dough) | Leavening Ability Ratio (90-Minute Floor Time/ 30-Minute Floor Time) |
| Inventive Dry Yeast (D66785) | 150 | 119 | 0.79 |
| Commercially Available Dry Yeast Saf-instant (Gold) | 147 | 29 | 0.20 |
| Commercially Available Dry Yeast Fermipan Brown | 126 | 38 | 0.30 |

As shown in Table 40, it can be seen that the leavening ability ratio expressing the floor resistance (leavening ability in the case of 90-minute floor time/leavening ability in the case of 30-minute floor time) of the inventive dry yeast (D92764) is 0.38 and that of the inventive dry yeast (D66785) is 0.79, while those of the commercially available dry yeasts are 0.30 or less, showing that the inventive dry yeasts have an excellent floor resistance.

In addition, the floor resistance at each point was determined by setting a frozen storage time period to 1 week, 2 weeks and 4 weeks. The results are shown in Figure 12. As is clear from Figure 12, in a dough having a sugar concentration of 25% by weight, the inventive dry yeasts showed a marked floor resistance in any of the frozen storage time periods of 1 week, 2 weeks and 4 weeks, as compared to those of the commercially available dry yeasts.

Example 20

The low-temperature sensitivity was determined for the inventive dry yeast D31735 by the method described in the above-mentioned (8) Low-Temperature Sensitivity. As the comparative controls, commercially available low-temperature sensitive compressed yeast Kaneka Yeast AL (manufactured by Kaneka Corporation) and 6 commercially available dry yeasts, Saf-instant (RED) (manufactured by Company S), Saf-instant (Gold) (manufactured by Company S), Mauripan low sugar (manufactured by Company BP), Bruggeman Blue (manufactured by Company BR), Fermipan RED (manufactured by Company D) and Fermipan Brown (manufactured by Company D) were used. The results are shown in Table 41.

Table 41

| Comparison of Low-Temperature Sensitivity of Inventive Dry Yeast and Commercially Available Dry Yeasts | | | |
|---|---|---|---|
| | Leavening Ability in Dough at 5°C (ml) | Leavening Ability in Dough at 30°C (ml) | Leavening Ability Ratio (Leavening Ability in Dough at 30°C/ Leavening Ability in Dough at 5°C) |
| Commercially Available Low-Temperature Sensitive Compressed yeast Kaneka Yeast AL | 165 | 169 | 1.02 |
| Inventive Dry Yeast (D31735) | 145 | 146 | 1.01 |
| Commercially Available Dry Yeast Saf-instant (RED) | 265 | 142 | 0.54 |
| Commercially Available Dry Yeast Saf-instant (Gold) | 245 | 158 | 0.64 |
| Commercially Available Dry Yeast Mauripan low sugar | 245 | 153 | 0.62 |

Table 41 (continued)

| Comparison of Low-Temperature Sensitivity of Inventive Dry Yeast and Commercially Available Dry Yeasts | | | |
|---|---|---|---|
| | Leavening Ability in Dough at 5°C (ml) | Leavening Ability in Dough at 30°C (ml) | Leavening Ability Ratio (Leavening Ability in Dough at 30°C/ Leavening Ability in Dough at 5°C) |
| Commercially Available Dry Yeast Bruggeman Blue | 250 | 134 | 0.54 |
| Commercially Available Dry Yeast Fermipan RED | 240 | 139 | 0.58 |
| Commercially Available Dry Yeast Fermipan Brown | 250 | 144 | 0.58 |

As is clear in Table 41, it can be seen that the leavening ability ratio in a dough at 30°C to the leavening ability in a dough at 5°C of the inventive dry yeast is 1.01, while those of the commercially available compressed yeast having low-temperature sensitivity are 1.02, showing that they have almost the same level of low-temperature sensitivity. On the other hand, any of the six commercially available dry yeasts has the ratio of 0.64 or less, so that it can be seen that the commercially available dry yeasts have impaired low-temperature sensitivity as compared to the dry yeast of the present invention.

Deposited Biological Materials

(1) Name and Addressee of Depository Authority

The International Patent Organism Depositary, National Institute of Advanced Industrial Science and Technology
Tsukuba Central 6, 1-1, Higashi 1-chome Tsukuba-shi, Ibaraki-ken, Japan (Zip code 305-8566)

(2) Deposited Microorganisms

1) *Saccharomyces cerevisiae* D75412

Original Date of Deposit: September 8, 2000
Date of Request for Transfer to International Deposit: August 1, 2001
Accession Number: FERM BP-7688

2) *Saccharomyces cerevisiae* D20946

Original Date of Deposit: September 8, 2000
Date of Request for Transfer to International Deposit: August 1, 2001
Accession Number: FERM BP-7684

3) *Saccharomyces cerevisiae* D46462

Original Date of Deposit: September 8, 2000
Date of Request for Transfer to International Deposit: August 1, 2001
Accession Number: FERM BP-7686

4) *Saccharomyces cerevisiae* D66785

Original Date of Deposit: September 8, 2000
Date of Request for Transfer to International Deposit: August 1, 2001
Accession Number: FERM BP-7687

5) *Saccharomyces cerevisiae* D92764

Original Date of Deposit: February 20, 2001
Date of Request for Transfer to International Deposit: August 1, 2001
Accession Number: FERM BP-7690

6) *Saccharomyces cerevisiae* D80921

Original Date of Deposit: September 8, 2000
Date of Request for Transfer to International Deposit: August 1, 2001
Accession Number: FERM BP-7689

*7) Saccharomyces cerevisiae* D31735

Original Date of Deposit: September 8, 2000
Date of Request for Transfer to International Deposit: August 1, 2001
Accession Number: FERM BP-7685

<u>INDUSTRIAL APPLICABILITY</u>

According to the present invention, there are provided an yeast having an excellent leavening ability in various kinds of dough from those containing no sugar to those having high-sugar concentration, and having drying tolerance, which is suitable for producing bread, especially suitable for producing bread made from frozen dough, specifically an yeast having a high leavening ability in high-sugar content dough to a super-high sugar content dough, and having drying tolerance; an yeast having a high leavening ability in a dough containing no sugar to a high-sugar content dough, and having drying tolerance; an yeast having a high leavening ability in a dough containing no sugar to a low-sugar content dough, and drying tolerance; an yeast having a high freezing tolerance and/or floor resistance in a medium sugar content dough to a high-sugar content dough, and having drying tolerance; an yeast having a high freezing tolerance and/or floor resistance in a dough containing no sugar to a high-sugar content dough, and having drying tolerance; an yeast having a high freezing tolerance and/or floor resistance in a dough containing no sugar to a low-sugar content dough, and having drying tolerance; and an yeast having low-temperature sensitivity, and having drying tolerance. Also, according to the present invention, there is provided a dry yeast produced by drying the above-mentioned yeast, which is excellent in preservability and storage stability, and capable of exhibiting a leavening ability of the same level as that of a raw yeast, especially suitable for producing bread made from frozen dough. Further, according to the present invention, there is provided a dough or a frozen dough, comprising the above-mentioned yeast or the above-mentioned dry yeast; and bread having excellent quality stability produced by using the dough.

**Claims**

1. An yeast having a leavening ability in a high-sugar content dough, and having drying tolerance.

2. The yeast according to claim 1, wherein the leavening ability in the form of a dry yeast, as expressed by an amount of gas generated at 30°C for 115 minutes in 85 g of a dough having a sugar concentration of 30% by weight (dry yeast: 1.5% by weight), is 200 ml or more.

3. The yeast according to claim 1 or 2, wherein the leavening ability in the form of a dry yeast, as expressed by an amount of gas generated at 30°C for 115 minutes in 85 g of a dough having a sugar concentration of 40% by weight (dry yeast: 1.5% by weight), is 70 ml or more.

4. An yeast having a leavening ability in a dough having a sugar concentration of 0 to 30% by weight, and having drying tolerance.

5. The yeast according to claim 4, wherein the leavening ability in the form of a dry yeast, as expressed by an amount of gas generated at 30°C for 85 minutes in 85 g of a dough having a sugar concentration of 0% by weight (dry yeast: 1% by weight), is 140 ml or more, and wherein the leavening ability in the form of a dry yeast, as expressed by an amount of gas generated at 30°C for 115 minutes in 85 g of a dough having a sugar concentration of 30% by weight (dry yeast: 1.5% by weight), is 200 ml or more.

6. The yeast according to any one of claims 1 to 5, wherein the leavening ability in the form of a dry yeast, as expressed by an amount of gas generated at 30°C for 120 minutes in 50 g of a dough after main kneading in the sugar-added sponge and dough process (sugar concentration: 28% by weight, dry yeast: 1.5% by weight), is 120 ml or more.

7. An yeast having a leavening ability in a dough having a sugar concentration of 0 to 5% by weight, and having drying tolerance.

8. The yeast according to claim 7, wherein the leavening ability in the form of a dry yeast, as expressed by an amount of gas generated at 30°C for 85 minutes in 85 g of a dough having a sugar concentration of 0% by weight (dry yeast: 1% by weight), is 220 ml or more.

9. The yeast according to claim 7 or 8, wherein the leavening ability in the form of a dry yeast, as expressed by an amount of gas generated at 30°C for 85 minutes in 85 g of a dough having a sugar concentration of 5% by weight (dry yeast: 1% by weight), is 160 ml or more.

10. An yeast having freezing tolerance and/or floor resistance in a dough having a sugar concentration of 10 to 30% by weight, and having drying tolerance.

11. The yeast according to claim 10, wherein the leavening ability in the form of a dry yeast, as expressed by an amount of gas generated at 38°C for 120 minutes in 20 g of a dough having a sugar concentration of 10% by weight (dry yeast: 2% by weight), is 90 ml or more in a dough after a 4-week frozen storage following a 60-minute floor time.

12. The yeast according to claim 11, wherein the yeast further has a ratio of leavening abilities before and after a 4-week frozen storage following a 60-minute floor time (after frozen storage/before frozen storage) of 0.50 or more.

13. The yeast according to claim 11 or 12, wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.20 or more.

14. The yeast according to claim 10, wherein the leavening ability in the form of a dry yeast, as expressed by an amount of gas generated at 38°C for 120 minutes in 20 g of a dough having a sugar concentration of 15% by weight (dry yeast: 2.5% by weight), is 70 ml or more in a dough after a 4-week frozen storage following a 60-minute floor time.

15. The yeast according to claim 14, wherein the yeast further has a ratio of leavening abilities before and after a 4-week frozen storage following a 60-minute floor time (after frozen storage/before frozen storage) of 0.40 or more.

16. The yeast according to claim 14 or 15, wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.20 or more.

17. The yeast according to claim 10, wherein the leavening ability in the form of a dry yeast, as expressed by an amount of gas generated at 38°C for 120 minutes in 20 g of a dough having a sugar concentration of 25% by weight (dry yeast: 3% by weight), is 50 ml or more in a dough after a 4-week frozen storage following a 90-minute floor time.

**18.** The yeast according to claim 17, wherein the yeast further has a ratio of leavening abilities before and after a 4-week frozen storage following a 90-minute floor time (after frozen storage/before frozen storage) of 0.60 or more.

**19.** The yeast according to claim 17 or 18, wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.70 or more.

**20.** An yeast having freezing tolerance and/or floor resistance in a dough having a sugar concentration of 0 to 30% by weight, and having drying tolerance.

**21.** The yeast according to claim 20, wherein the leavening ability in the form of a dry yeast, as expressed by an amount of gas generated at 38°C for 120 minutes in 20 g of a dough having a sugar concentration of 0% by weight (dry yeast: 2% by weight), is 100 ml or more in a dough after a 4-week frozen storage following a 60-minute floor time.

**22.** The yeast according to claim 21, wherein the yeast further has a ratio of leavening abilities before and after a 4-week frozen storage following a 60-minute floor time (after frozen storage/before frozen storage) of 0.88 or more.

**23.** The yeast according to claim 21 or 22, wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.80 or more.

**24.** The yeast according to any one of claims 20 to 23, wherein the leavening ability in the form of a dry yeast, as expressed by an amount of gas generated at 38°C for 120 minutes in 20 g of a dough having a sugar concentration of 5% by weight (dry yeast: 2% by weight), is 70 ml or more in a dough after a 4-week frozen storage following a 60-minute floor time.

**25.** The yeast according to claim 24, wherein the yeast further has a ratio of leavening abilities before and after a 4-week frozen storage following a 60-minute floor time (after frozen storage/before frozen storage) of 0.40 or more.

**26.** The yeast according to claim 24 or 25, wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.50 or more.

**27.** The yeast according to any one of claims 20 to 26, wherein the leavening ability in the form of a dry yeast, as expressed by an amount of gas generated at 38°C for 120 minutes in 20 g of a dough having a sugar concentration of 10% by weight (dry yeast: 2% by weight), is 90 ml or more in a dough after a 4-week frozen storage following a 60-minute floor time.

**28.** The yeast according to claim 27, wherein the yeast further has a ratio of leavening abilities before and after a 4-week frozen storage following a 60-minute floor time (after frozen storage/before frozen storage) of 0.50 or more.

**29.** The yeast according to claim 27 or 28, wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.20 or more.

**30.** The yeast according to any one of claims 20 to 29, wherein the leavening ability in the form of a dry yeast, as expressed by an amount of gas generated at 38°C for 120 minutes in 20 g of a dough having a sugar concentration of 25% by weight (dry yeast: 3% by weight), is 125 ml or more in a dough after a 4-week frozen storage following a 60-minute floor time, and is 50 ml or more in a dough after a 4-week frozen storage following a 90-minute floor time.

**31.** The yeast according to claim 30, wherein the yeast further has a ratio of leavening abilities before and after a 4-week frozen storage following a 60-minute floor time (after frozen storage/before frozen storage) of 0.70 or more, and a ratio of leavening abilities before and after a 4-week frozen storage following a 90-minute floor time (after frozen storage/before frozen storage) of 0.30 or more.

**32.** The yeast according to claim 30 or 31, wherein the yeast further has a ratio of a leavening ability after a 4-week frozen storage following a 90-minute floor time to a leavening ability after a 4-week frozen storage following a 30-minute floor time (90-minute floor time/30-minute floor time) of 0.35 or more.

**33.** An yeast having freezing tolerance and/or floor resistance in a dough having a sugar concentration of 0 to 3% by weight, and having drying tolerance.

**34.** The yeast according to claim 33, wherein the leavening ability in the form of a dry yeast, as expressed by an amount of gas generated at 38°C for 120 minutes in 20 g of a dough having a sugar concentration of 0% by weight (dry yeast: 2% by weight), is 100 ml or more in a dough after a 4-week frozen storage following a 60-minute floor time.

**35.** The yeast according to claim 34, wherein the yeast further has a ratio of leavening abilities before and after a 4-week frozen storage following a 60-minute floor time (after frozen storage/before frozen storage) of 0.88 or more.

**36.** The yeast according to claim 34 or 35, wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.80 or more.

**37.** The yeast according to any one of claims 33 to 36, wherein the leavening ability in the form of a dry yeast, as expressed by an amount of gas generated at 38°C for 120 minutes in 20 g of a dough having a sugar concentration of 3% by weight (dry yeast: 2% by weight), is 50 ml or more in a dough after a 4-week frozen storage following a 60-minute floor time.

**38.** The yeast according to claim 37, wherein the yeast further has a ratio of leavening abilities before and after a 4-week frozen storage following a 60-minute floor time (after frozen storage/before frozen storage) of 0.40 or more.

**39.** The yeast according to claim 37 or 38, wherein the yeast further has a ratio of a leavening ability after a 4-week frozen storage following a 60-minute floor time to a leavening ability after a 4-week frozen storage following a 0-minute floor time (60-minute floor time/0-minute floor time) of 0.35 or more.

**40.** An yeast having low-temperature sensitivity, and having drying tolerance.

**41.** The yeast according to any one of claims 1 to 40, wherein the yeast has a remaining leavening ability ratio [ratio of leavening abilities before and after drying (after drying/before drying)] of 0.70 or more.

**42.** The yeast according to any one of claims 1 to 3 and 41, which is *Saccharomyces cerevisiae* D75412 (FERM BP-7688).

**43.** The yeast according to any one of claims 4 to 6 and 41, which is *Saccharomyces cerevisiae* D20946 (FERM BP-7684).

**44.** The yeast according to any one of claims 7 to 9 and 41, which is *Saccharomyces cerevisiae* D46462 (FERM BP-7686).

**45.** The yeast according to any one of claims 10 to 19 and 41, which is *Saccharomyces cerevisiae* D66785 (FERM BP-7687).

**46.** The yeast according to any one of claims 20 to 32 and 41, which is *Saccharomyces cerevisiae* D92764 (FERM BP-7690).

**47.** The yeast according to any one of claims 33 to 39 and 41, which is *Saccharomyces cerevisiae* D80921 (FERM BP-7689).

**48.** The yeast according to any one of claims 40 and 41, which is *Saccharomyces cerevisiae* D31735 (FERM BP-7685).

**49.** The yeast according to any one of claims 1 to 48, wherein the yeast is a dry yeast.

**50.** The yeast according to any one of claims 10 to 49, which is usable for a frozen dough.

**51.** A dough comprising the yeast of any one of claims 1 to 50.

**52.** Bread produced by using the dough of claim 51.

# FIG. 1

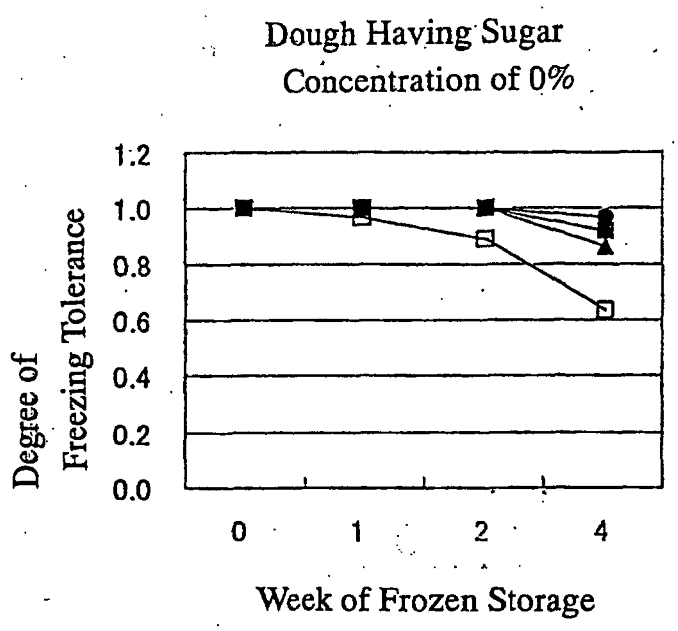
Dough Having Sugar
Concentration of 0%
Degree of
Freezing Tolerance
1.2
1.0
0.8
0.6
0.4
0.2
0.0
0
1
2
4
Week of Frozen Storage

# FIG. 2

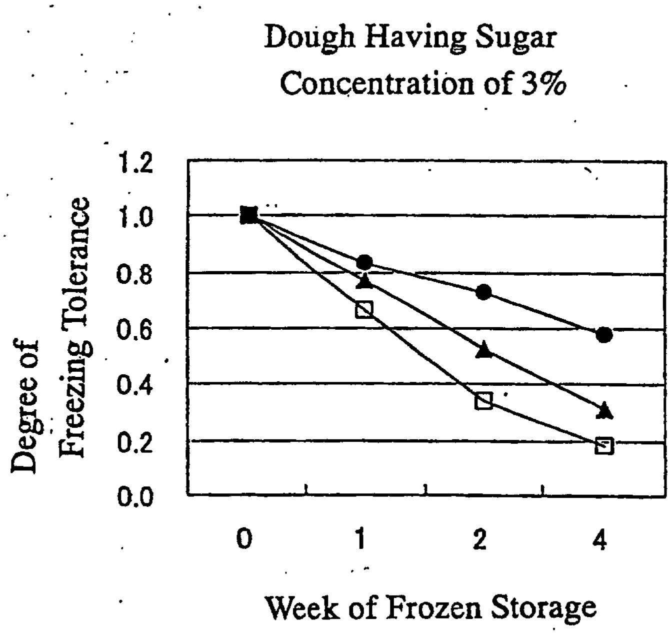
Dough Having Sugar
Concentration of 3%
Degree of
Freezing Tolerance
1.2
1.0
0.8
0.6
0.4
0.2
0.0
0
1
2
4
Week of Frozen Storage

FIG. 3

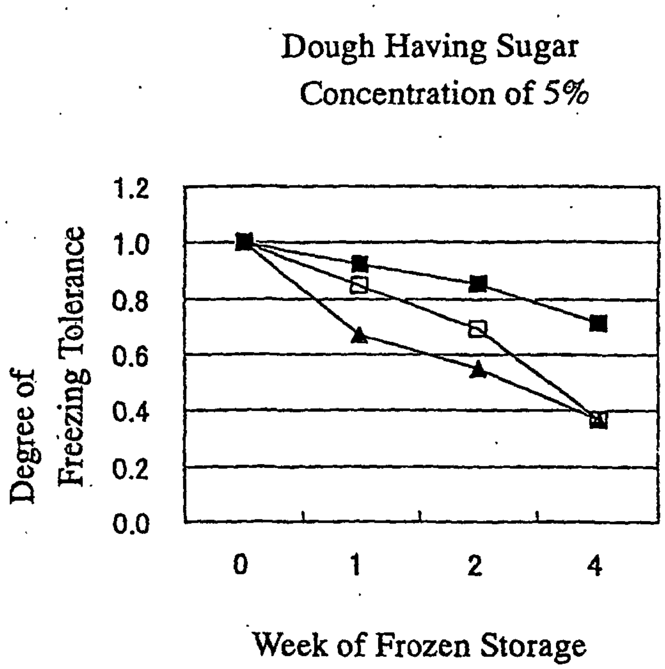
Dough Having Sugar
Concentration of 5%
1.2
1.0
0.8
0.6
0.4
0.2
0.0
Degree of
Freezing Tolerance
0
1
2
4
Week of Frozen Storage

FIG. 4

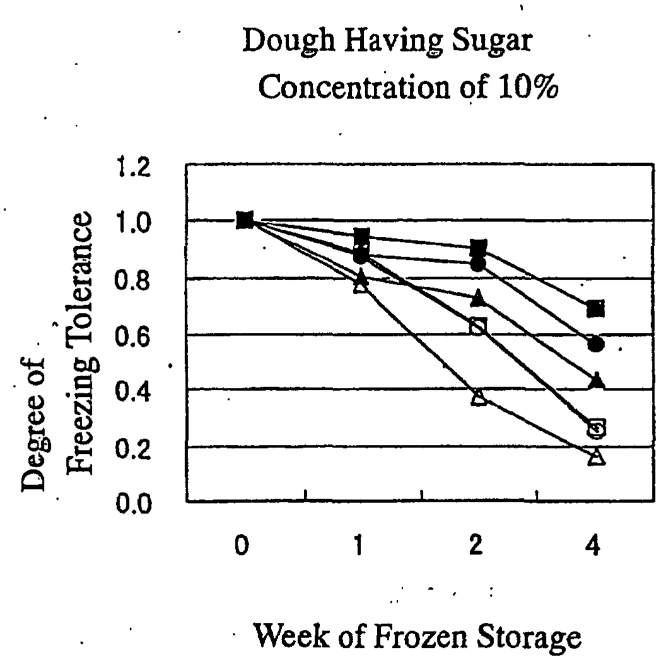

Dough Having Sugar
Concentration of 10%
1.2
1.0
0.8
0.6
0.4
0.2
0.0
Degree of
Freezing Tolerance
0
1
2
4
Week of Frozen Storage

# FIG. 5

Dough Having Sugar Concentration of 15%

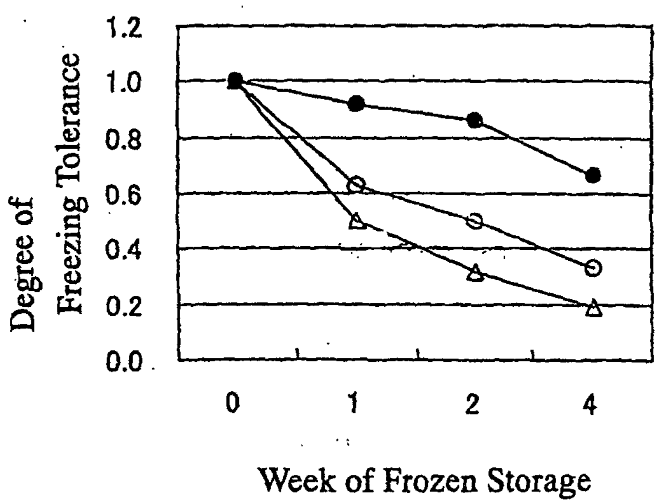

FIG. 6

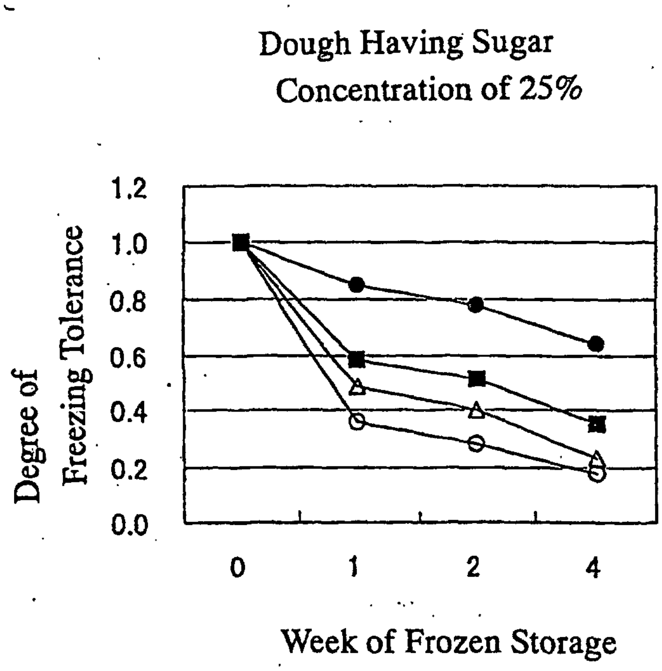
Dough Having Sugar Concentration of 25%
Degree of Freezing Tolerance
1.2
1.0
0.8
0.6
0.4
0.2
0.0
0
1
2
4
Week of Frozen Storage

# FIG.7

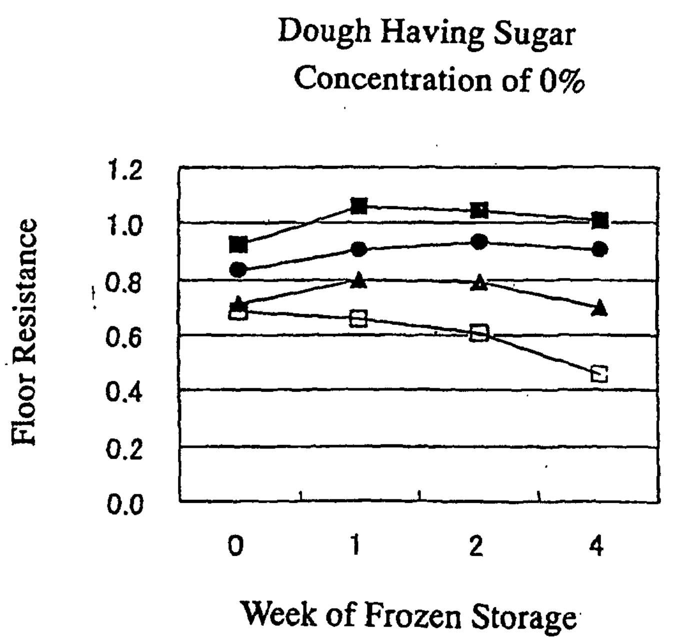
Dough Having Sugar
Concentration of 0%
Floor Resistance
1.2
1.0
0.8
0.6
0.4
0.2
0.0
0
1
2
4
Week of Frozen Storage

# FIG.8

Dough Having Sugar Concentration of 3%

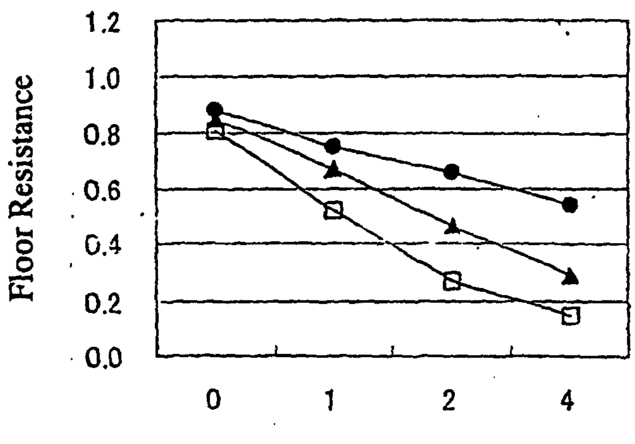

# FIG.9

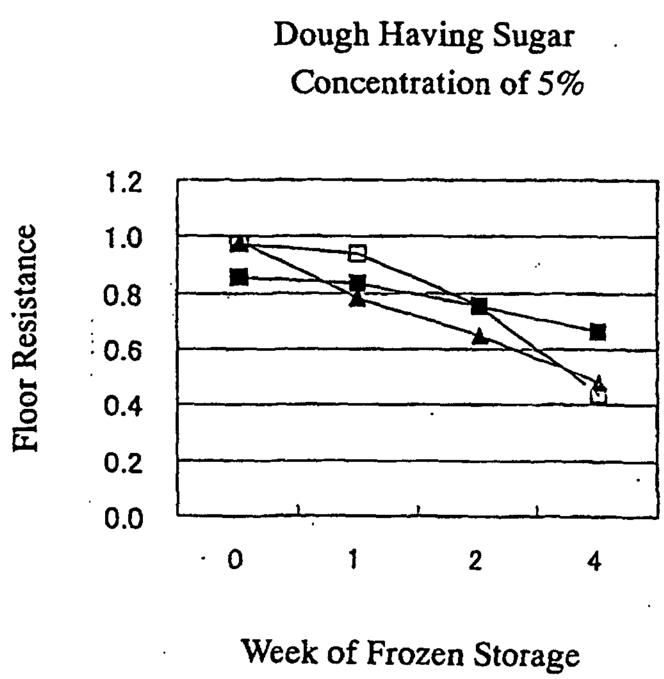
Dough Having Sugar
Concentration of 5%
Floor Resistance
1.2
1.0
0.8
0.6
0.4
0.2
0.0
0
1
2
4
Week of Frozen Storage

# FIG.10

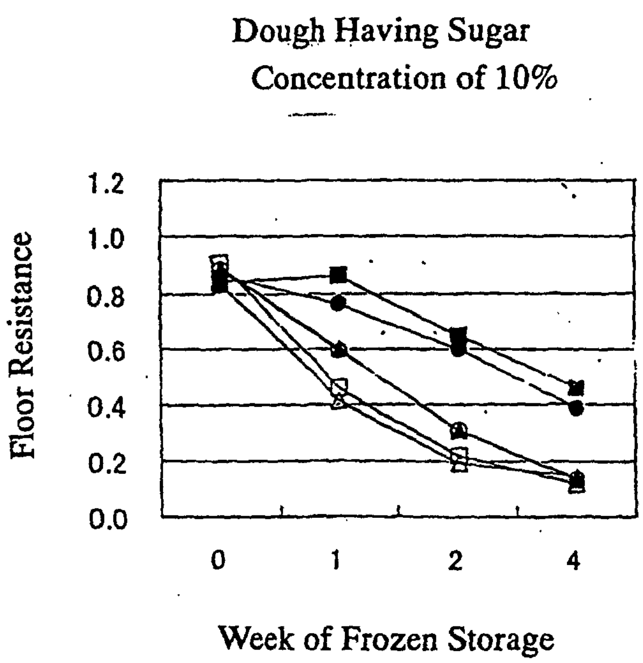
Dough Having Sugar
Concentration of 10%
Floor Resistance
1.2
1.0
0.8
0.6
0.4
0.2
0.0
0
1
2
4
Week of Frozen Storage

# FIG.11

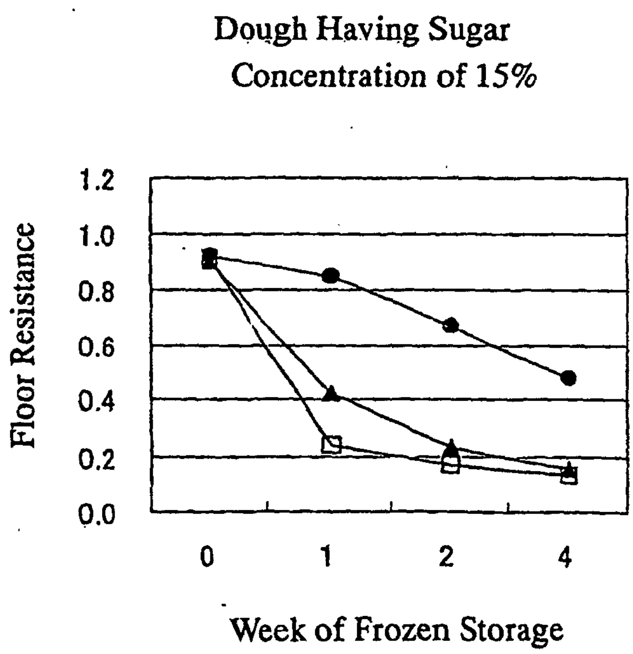
Dough Having Sugar
Concentration of 15%
Floor Resistance
1.2
1.0
0.8
0.6
0.4
0.2
0.0
0
1
2
4
Week of Frozen Storage

FIG.12

Dough Having Sugar Concentration of 25%

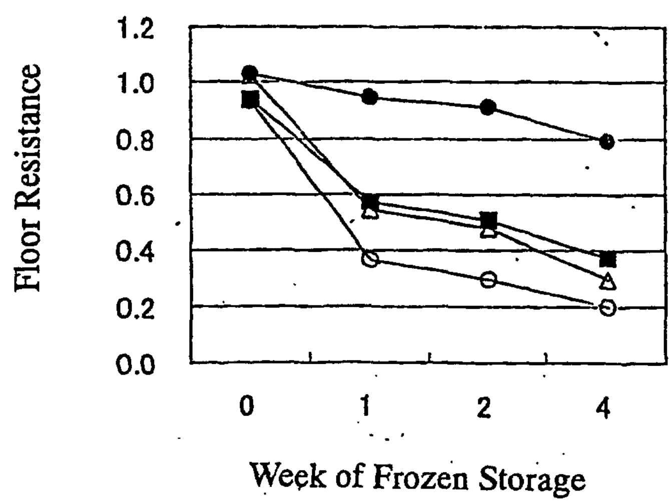

## INTERNATIONAL SEARCH REPORT

International application No.
PCT/JP01/08668

A. CLASSIFICATION OF SUBJECT MATTER
Int.Cl[7] C12N1/18, A21D8/04 // (C12N1/18, C12R1:865)

According to International Patent Classification (IPC) or to both national classification and IPC

B. FIELDS SEARCHED

Minimum documentation searched (classification system followed by classification symbols)
Int.Cl[7] C12N1/16-1/19

Documentation searched other than minimum documentation to the extent that such documents are included in the fields searched

Electronic data base consulted during the international search (name of data base and, where practicable, search terms used)
BIOSIS(DIALOG),WPI(DIALOG),JICST FILE(JOIS)

C. DOCUMENTS CONSIDERED TO BE RELEVANT

| Category* | Citation of document, with indication, where appropriate, of the relevant passages | Relevant to claim No. |
|---|---|---|
| X<br>A | CA 1105318 A (LESAFFRE ET CIE.),<br>21 July, 1981 (21.07.81),<br>& EP 8554 A2 & BE 878070 A<br>& FR 2433541 A & DE 2962809 G | 1<br>2-39,41-52 |
| A | EP 1036841 A1 (ORIENTAL YEAST CO., LTD.),<br>20 September, 2000 (20.09.00),<br>& CA 2300436 A1 & JP 2000-262275 A<br>& JP 2000-279165 A | 1-39,41-52 |
| A | JP 10-191964 A (Kanegafuchi Chem. Ind. Co., Ltd.),<br>28 July, 1998 (28.07.98)<br>(Family: none) | 1-39,41-52 |
| A | JP 9-149785 A (Nippon Beet Sugar Mfg. Co., Ltd.),<br>10 June, 1997 (10.06.97),<br>(Family: none) | 1-39,41-52 |
| A | EP 388262 A1 (NATIONAL FOOD RESEARCH INSTITUTE, MINISTRY OF AGRICULTURE, FORESTRY AND FISHERIES)<br>19 September, 1990 (19.09.90), | 1-39,41-52 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 January, 2002 (09.01.02) | 19 February, 2002 (19.02.02) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/08668

C (Continuation). DOCUMENTS CONSIDERED TO BE RELEVANT

| Category* | Citation of document, with indication, where appropriate, of the relevant passages | Relevant to claim No. |
|---|---|---|
| | & JP 2-238876 A & JP 6-70673 A<br>& CA 2012090 A & FI 9001267 A<br>& FI 97068 B & AU 9051261 A<br>& US 5352606 A & DE 69012860 E | |
| A | EP 451896 A1 (GIST-BROCADES N.V.),<br>16 October, 1991 (16.10.91)<br>& AU 9173782 A & AU 636022 B<br>& NO 9101232 A & CA 2039377 A<br>& FI 9101500 A & PT 97185 A<br>& ZA 9102382 A & EP 507009 A1<br>& JP 5-184353 A & NZ 237604 A<br>& US 5312909 A & DE 69116413 E<br>& IL 97692 A | 1-39,41-52 |

Form PCT/ISA/210 (continuation of second sheet) (July 1992)

**INTERNATIONAL SEARCH REPORT**

International application No.
PCT/JP01/08668

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

(See extra sheet.)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Claims 1 to 39, 41 (the part relating to claims 1 to 39), 42 to 48 and 49 to 52 (the parts relating to claims 1 to 39)

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP01/08668

Continuation of Box No.II of continuation of first sheet(1)

The requirement of unity of invention in international application (PCT Rule 13.1) shall be fulfilled only when there is a technical relationship in a group of claimed inventions involving one or more of the same or corresponding special technical features. The expression "special technical features" shall mean those technical features that define a contribution which each of the claimed inventions, considered as a whole, makes over the prior art (PCT Rule 13.2). The determination whether a group of inventions is so linked as to form a general inventive concept shall be made without regard to whether the inventions are claimed in separate claims or as alternatives within a single claim (PCT Rule 13.3).

In the claims of the present case:

(1) the inventions as set forth in claims 1 to 39, 41 (the part relating to claims 1 to 39), 42 to 48 and 49 to 52 (the parts relating to claims 1 to 39); and

(2) the inventions as set forth in claims 40, 41 (the part relating to claim 40) and 49 to 52 (the parts relating to 40);

have a technical matter in common of "yeast having a drying-resistance". However, there had been well known "yeasts having a drying-resistance" without citing documents. Thus, it can be concluded that there is no "special technical feature" common to both of the groups of inventions as set forth in the above claims.

Such being the case, the claims involve two different groups of inventions (1) and (2) as mentioned above.

Form PCT/ISA/210 (extra sheet) (July 1992)